# EUROPÄISCHE PATENTSCHRIFT

(11) **EP 3 351 290 B1**
(45) Veröffentlichungstag und Bekanntmachung des Hinweises auf die Patenterteilung: **26.06.2019**
(21) Anmeldenummer: 17152617.1
(22) Anmeldetag: 23.01.2017
(51) Int. Cl.: A61N 1/375

(54) **ELEKTRISCHE DURCHFÜHRUNG MIT KONTAKTELEMENT UND VERFAHREN ZUR HERSTELLUNG**
ELECTRIC FEEDTHROUGH WITH CONTACT ELEMENT AND METHOD OF MANUFACTURING
TRAVERSÉE ÉLECTRIQUE COMPRENANT UN ÉLÉMENT DE CONTACT ET PROCÉDÉ DE FABRICATION

(43) Veröffentlichungstag der Anmeldung: 25.07.2018
(73) Patentinhaber: Heraeus Deutschland GmbH & Co. KG, 63450 Hanau (DE)
(72) Erfinder: Dittmer, Robert, 63450 Hanau (DE); Krüger, Frank, 61130 Nidderau (DE)
(74) Vertreter: Heraeus IP

(56) Entgegenhaltungen:
- EP-A1- 3 058 984
- US-A1- 2011 190 885
- US-A1- 2012 193 118
- US-A1- 2012 194 981

## Beschreibung

Die Erfindung betrifft eine elektrische Durchführung für eine medizinisch implantierbare Vorrichtung. Ferner betrifft die Erfindung ein Verfahren zum Aufbringen von Kontaktelementen auf elektrische Durchführungen.

Die DE 697 297 19 T2 beschreibt eine elektrische Durchführung für eine aktive, implantierbare, medizinische Vorrichtung - auch als implantierbare Vorrichtung oder Therapiegerät bezeichnet. Derartige elektrische Durchführungen dienen dazu, eine elektrische Verbindung zwischen einem hermetisch abgeschlossenen Inneren und einem Äußeren des Therapiegerätes herzustellen. Bekannte implantierbare Therapiegeräte sind Herzschrittmacher oder Defibrillatoren, die üblicherweise ein hermetisch dichtes Metallgehäuse aufweisen, welches auf einer Seite mit einem Anschlusskörper, auch Header oder Kopfteil genannt, versehen ist. Der Anschlusskörper dient der Verbindung mit Elektrodenleitungen. Die Anschlussbuchse weist elektrische Kontakte auf, um die Elektrodenleitungen elektrisch mit der Steuerelektronik im Inneren des Gehäuses des implantierbaren Therapiegerätes zu verbinden. Dabei sind einerseits elektrische Verbindungen zwischen dem Anschlusskörper und der äußeren Seite der elektrischen Durchführung vorgesehen. Andererseits sind elektrische Verbindungen zwischen der inneren Seite der Durchführung und der Steuerelektronik vorgesehen. Das Dokument US-A-2011/190885 offenbart eine Durchführung gemäß Oberbegriff des Anspruchs 1.

Eine wesentliche Voraussetzung für eine elektrische Durchführung ist die hermetische Dichtigkeit gegenüber einer Umgebung. Folglich müssen in einen elektrisch isolierenden Grundkörper eingebrachte Leitungselemente, über welche die elektrische Signale laufen, spaltfrei in den isolierenden Grundkörper eingebracht werden. Dabei hat sich als vorteilhaft erwiesen, die Leitungselemente als Cermet auszugestalten. Cermets sind Verbundwerkstoffe aus pulverförmigem Metall und einer oder mehreren Keramiken. Cermets ermöglichen die Herstellung einer unmittelbaren stoffschlüssigen Verbindung zwischen dem Leitungselement und dem umgebenden isolierenden keramischen Grundkörper der Durchführung durch co-sintern. Werden massive metallische Leitungsdrähte statt Cermets verwendet, müssen diese in aufwendigen Verfahren in die Keramik eingebracht werden, um eine hermetisch dichte Verbindung zu erzeugen. Dabei kommen Verfahren zum Einsatz, die eine Metallisierung der Keramik in der Durchgangsöffnung und Lötprozesse unter Verwendung von Lotringen erfordern. Insbesondere die Metallisierung in der Durchgangsöffnung hat sich als schwierig aufzubringen herausgestellt.

Andererseits verfügen Cermet-Verbundwerkstoffe über einen begrenzten Metallanteil. Dadurch lassen sich in vielen Fällen keine direkten ausreichend stabilen Verbindungen mit Hilfe von Metalldrähten zwischen der freiliegenden Fläche eines Cermet-Leitungselements und dem Anschlusskörper auf der Außenseite beziehungsweise der Steuerelektronik auf der Innenseite eines Gehäuses herstellen. Die verwendeten Drähte verfügen oft nur über einen Durchmesser von wenigen Mikrometern. In der Cermet-Oberfläche des freiliegenden Bereichs des Leitungselements weisen die Metallpartikel in Abhängigkeit von der verwendeten Partikelgröße des Metall- bzw. Keramikpulvers in vielen Fällen einen so großen Abstand voneinander auf, dass keine zuverlässige direkte Kontaktierung mit einem Metalldraht möglich ist. Daher ist in solchen Fällen eine Verbindungsschicht, auch Kontaktelement, erforderlich, die eine ausreichend zugfeste Verbindung zwischen dem Cermet-Leitungselement und einem Metalldraht sicherstellt. Das Kontaktelement ist zwischen dem Leitungselement der elektrischen Durchführung und dem Metalldraht angeordnet. Üblicherweise besteht das Kontaktelement aus einem geeigneten Metall oder einer geeigneten Metall-Legierung.

Solche Kontaktelemente können mit bekannten Verfahren wie Druckverfahren oder PVD-Verfahren erzeugt werden.

Bekannte Druckverfahren sind Tampondruck und Siebdruck. Wird die Verbindungsschicht über ein Druckverfahren aufgebracht, so kann beispielsweise eine Druckpaste verwendet werden, die mindestens ein leitendes Material enthält. Häufig enthält die Paste zusätzlich noch einen oder mehrere organische Bindemittel, wie beispielsweise eine Alkylcellulose. Beim Entfernen des Bindemittels durch Erhitzen wird eine Metallschicht erhalten, die in der Regel über eine starke Porosität verfügt.

Ein bekanntes PVD-Verfahren ist das als Kathodenzerstäubungsprozess bekannte Sputtern. Dabei werden durch Beschuss mit energiereichen Ionen aus einem Festkörper Atome herausgeschlagen, welche sich anschließend auf dem Substrat, in diesem Fall dem Leitungselement, als Schicht niederlegen. So erzeugte Schichte verfügen oft über eine niedrige Porosität. Die Erzeugung von PVD generierten Schichten mit hohen Schichtdicken ist wirtschaftlich nicht rentabel.

Sowohl Druckverfahren als auch PVD-Verfahren sind maskengebende Verfahren. Sollen nur bestimmte Bereiche eines Substrates, hier die freiliegenden Flächen des Leitungselements, mit dem Kontaktelement beschichtet werden, so ist eine Maskierung der umliegenden Bereiche erforderlich. Maskierende Verfahren stoßen dort häufig an ihre Grenzen, wo sehr kleine Flächen oder dicht nebeneinander liegende Flächen beschichtet werden sollen. Dies ist aufgrund der fortschreitenden Miniaturisierung und immer komplexer werdenden Vorrichtungen in der Medizintechnik oft mit Problemen verbunden. Weiterhin ist die Beschichtung von gekrümmten Flächen den Druckverfahren nicht zugänglich. Wie oben erwähnt führen Druckverfahren oft zu hochporösen Verbindungsschichten oder Kontaktelementen, die keine zugfeste Verbindung mit einem Metalldraht ermöglichen. Sind größere Schichtdicken für das Kontaktelement erforderlich, sind PVD-Verfahren wirtschaftlich nicht rentabel. Allgemein sind maskengebende Verfahren bei sehr kleinen und/oder dicht nebeneinander liegenden zu beschichtenden Flächen nicht geeignet.

Allgemein ist es eine Aufgabe der vorliegenden Erfindung, die vorgenannten Nachteile des Standes der Technik wenigstens teilweise zu überwinden. Die Aufgabe der Erfindung ist insbesondere darin zu sehen, eine elektrische Durchführung mit einem die freiliegende Fläche eines Cermet-Leitungselementes überlagernden Kontaktelements bereitzustellen, wobei das Kontaktelement derart ausgestaltet ist, dass es kleinflächig erzeugt werden kann, die freiliegende Fläche des Leitungselements möglichst randlos überdeckt, und eine so niedrige Porosität aufweist, dass eine zugfeste Verbindung mit einem Metalldraht oder einer anderen metallhaltigen drahtähnlichen Struktur erzeugt werden kann. Eine weitere Aufgabe der Erfindung liegt darin ein Verfahren bereitzustellen, das die Herstellung von elektrischen Durchführungen mit Kontaktelementen ermöglicht, welches die oben genannten Kriterien erfüllt. Insbesondere soll das Verfahren ermöglichen, Durchführungen mit Kontaktelementen auf gekrümmten Oberflächen bereitzustellen. Gleichzeitig muss das Verfahrene ermöglichen Kontaktelemente auf dicht nebeneinander liegenden Leitungselementen zu erzeugen, ohne dass sich die Kontaktelemente untereinander berühren.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie, insbesondere der erfindungsgemäßen elektrischen Durchführung und des erfindungsgemäßen Verfahrens, sind ebenso bevorzugt für gleichnamige oder entsprechende Bestandeile der jeweils anderen erfindungsgemäßen Kategorie. Die Ausdrücke "aufweisend", "umfassend" oder "beinhaltend" usw. schließen nicht aus, dass weitere Elemente, Inhaltsstoffe usw. enthalten sein können. Der unbestimmte Artikel "ein" schließt nicht aus, dass eine Mehrzahl vorhanden sein kann.

In einem ersten Aspekt der Erfindung wird eine elektrische Durchführung für eine medizinisch implantierbare Vorrichtung vorgeschlagen, umfassend einen elektrisch isolierenden Grundkörper und ein elektrisches Leitungselement, wobei das Leitungselement ein Cermet beinhaltet, und wobei der Grundkörper und das Leitungselement durch eine stoffschlüssige gesinterte Verbindung verbunden sind, so dass das Leitungselement hermetisch gegen den Grundkörper abgedichtet ist; sich das Leitungselement von einer ersten Oberfläche des Grundkörpers durch den Grundkörper hindurch zu einer zweiten Oberfläche des Grundkörpers erstreckt, wobei das Leitungselement einen ersten elektrisch leitfähigen Bereich innerhalb der ersten Oberfläche des Grundkörpers und einen zweiten elektrisch leitfähigen Bereich innerhalb der zweiten Oberfläche des Grundkörpers aufweist, und wenigstens einer der elektrisch leitfähigen Bereiche durch ein schichtartiges Kontaktelement, das ein Metall beinhaltet, wenigstens teilweise überlagert ist, so dass das Leitungselement über das Kontaktelement elektrisch leitend verbindbar ist, wobei das Kontaktelement eine elektrochemisch erzeugte Schicht ist und das Kontaktelement eine poröse Struktur aufweist, wobei die Porosität des Kontaktelements nicht mehr als 20 % beträgt.

Da das Kontaktelement eine elektrochemisch erzeugte Schicht ist, ist kein maskengebendes Verfahren erforderlich. Die elektrochemische Abscheidung zur Erzeugung der Schicht findet nur in einem Bereich statt, in dem tatsächlich elektrisch leitfähige Partikel vorhanden sind. Dies ist bei der elektrochemischen Abscheidung nur an den freiliegenden Oberflächen des Leitungselements, das heißt am elektrisch leitfähigen Bereich innerhalb des isolierenden Grundkörpers der Fall. Dabei wurde überraschend gefunden, dass trotz der durch den Cermet bedingten Mischung aus Metallpartikeln und Keramikpartikeln und der räumlichen Trennung dieser Partikel innerhalb des Verbundes ein Kontaktelement erzeugt werden kann, welches ausreichend dicht und ausreichend fest haftend auf dem Leitungselement ist. Dabei verfügt das Kontaktelement über eine Porosität von nicht mehr als 20 % und ist daher für das Anbringen von Metalldrähten geeignet. Dadurch werden Verbindungen mit ausreichender Zugfestigkeit erhalten. Zum Anbringen der Metalldrähte an das Kontaktelement eignen sich Schweißverfahren wie beispielsweise Widerstandsschweißen, Ultraschallschweißen, Laserschweißen oder ein Mikroprozess-Schweißverfahren.

Bei dem Mikroprozess-Schweißverfahren wirkt beispielsweise ein zeitlich begrenzter Druck für mehrere Minuten bis Stunden auf die Schnittstelle von Draht zum Kontaktelement. Darüber hinaus oder alternativ kann bei erhöhter Temperatur gearbeitet werden, vorzugsweise im Bereich von 180-220 °C. Weiterhin oder alternativ kann das Mikroprozess-Schweißverfahren unter Einfluss von Ultraschallwellen durchgeführt werden. Durch die so behandelten Materialien des Drahtes und des Kontaktelements können keil- bzw. kugelförmige stoffschlüssige Verbindungen erreicht werden. Vorzugsweise werden Drähte mit rundem Querschnitt verwendet.

Ebenfalls geeignet sind aus der Aufbau- und Verbindungstechnik bekannte Drahtbondverfahren wie Ball-Wedge Bonden oder Wedge-Wedge-Bonden.

Da das Kontaktelement eine elektrochemisch erzeugte Schicht ist, ist das schichtartige Kontaktelement mit der beschichteten Fläche des Kontaktelements nahezu deckungsgleich. Das schichtartige Kontaktelement überdeckt die leitfähige Fläche des Leitungselements also randlos oder nahezu randlos. Erst bei größeren Schichtdicken ist mit einem Wachstum der Schicht in radialer oder lateraler Richtung zu rechnen.

Das Kontaktelement ist eine elektrochemisch erzeugte Schicht, die durch elektrochemische Abscheidung erhalten wird. Unter "elektrochemischer Abscheidung" werden alle Verfahren verstanden, die zu einer Abscheidung eines Metalls aus einem elektrolytischen Bad auf einer metallhaltigen leitfähigen Oberfläche führen. Bei der Abscheidung auf dieser leitfähigen Oberfläche werden Metall-Kationen zum elementaren Metall reduziert. Der zu beschichtende Bereich stellt die Kathode dar. Üblicherweise findet die Abscheidung in einer rein wässrigen Umgebung statt, wobei die Metallkationen und Gegenionen (Anionen) in Wasser gelöst vorliegen. Es muss sich jedoch nicht um eine rein wässrige Umgebung handeln. In bestimmten Fällen kann die Anwesenheit von organischen Lösungsmitteln von Vorteil sein. Dabei kann von außen ein elektrischer Strom angelegt sein. Es sind jedoch auch stromlose Verfahren denkbar, bei denen die Abscheidung durch Auswahl eines geeigneten Redoxsystems spontan abläuft, ohne dass von außen ein elektrischer Strom angelegt werden muss. Insbesondere kommen in diesen Fällen Reduktionsverfahren zum Einsatz, bei denen zum Elektrolyt ein Reduktionsmittel zugegeben wird. Dabei führt die Oxidation des Reduktionsmittels unter Abgabe von Elektronen zu einer Reduktion der Metall-Kationen. Um eine gezielte Abscheidung aus derartigen Lösungen zu ermöglichen, darf die Metallabscheidung unter Verwendung eines Reduktionsmittels nur unter dem katalytischen Einfluss der zu beschichtenden Oberfläche des Kontaktelements ablaufen, da sonst eine unspezifische Abscheidung aller Oberflächen stattfände. Zur Aktivierung können palladiumhaltige Lösungen verwendet werden, welche die Oberfläche des Leitungselements bekeimen, so dass diese Oberfläche anschließend beispielsweise in einem Nickelbad katalytisch wirksam wird.

Die Porosität des elektrisch leitenden Kontaktelements stellt das Verhältnis von Hohlraumvolumen des Kontaktelements zum Gesamtvolumen des Kontaktelements dar.

Beispielsweise hat ein Kontaktelement mit einem Gesamtvolumen von 1 mm³ und einem Hohlraumvolumen von 0,15 mm³ eine Porosität von 15 %. Bevorzugt hat das Kontaktelement eine Porosität von mindestens 0,1 %. Die Prozent-Angabe der Porosität ist eine Angabe in Volumen-Prozenten (Vol.-%).

Das elektrisch leitende Kontaktelement hat die Eigenschaft einerseits eine feste und mechanisch belastbare Verbindung zu dem Leitungselement herzustellen, andererseits eine einfache aber mechanisch belastbare Verbindung zu weiteren elektrisch leitenden Elementen herzustellen, die an das Leitungselement angeschlossen werden sollen. Da das Leitungselement zumindest zu einem Teil aus einem Cermet hergestellt ist und beispielsweise zu einem Teil keramische Bestandteile aufweist, ist eine Verbindung zu weiteren elektrisch leitenden Materialien wie oben beschrieben nicht ohne weiteres möglich. Deshalb ist es vorteilhaft, von der elektrischen Durchführung zu elektrisch leitenden Materialien, wie beispielsweise einem Draht, der an das Leitungselement angeschlossen werden soll ein Kontaktelement einzufügen, das eine leicht bereitzustellende und feste Verbindung mit dem Leitungselement einerseits und beispielsweise dem Draht andererseits ermöglicht.

Das elektrisch leitende Kontaktelement schließt sich in der Regel an eine drahtähnliche Struktur an. Hierbei ist es bevorzugt, dass das Kontaktelement über die drahtähnliche Struktur mit einem oder mehreren Elementen der elektronischen Einheit einer implantierbaren Vorrichtung verbunden ist. Unter einer drahtähnlichen Struktur kann ein Draht oder eine geprägte Drahtstruktur verstanden werden. Die drahtähnliche Struktur ist befähigt, elektrischen Strom zu leiten. Dabei sollte ein Widerstand in einem Bereich von 0,1 bis 0,5 Ohm·mm²/m nicht überschritten werden. Darüber hinaus sollte die drahtähnliche Struktur eine Verbindung zwischen dem Leitungselement der elektrischen Durchführung und einer weiteren elektrischen Vorrichtung, wie beispielsweise einem weiteren Cermet, einer Batterie oder einer sonstigen elektrischen Einheit ermöglichen.

Als "Cermet" wird ein Verbundwerkstoff aus einem oder mehreren keramischen Werkstoffen in mindestens einer metallischen Matrix oder ein Verbundwerkstoff aus einem oder mehreren metallischen Werkstoffen in mindestens einer keramischen Matrix oder beides bezeichnet. Zur Herstellung eines Cermets kann beispielsweise ein Gemisch aus mindestens einem keramischen Pulver und mindestens einem metallischen Pulver verwendet werden. Dieses Gemisch kann mit mindestens einem Bindemittel und optional mindestens einem Lösungsmittel versetzt werden, um einen formbaren Grünkörper zu erhalten. Das Bindemittel und ggf. das Lösungsmittel werden beim sogenannten Entbindern später thermisch beziehungsweise durch Abdampfen vollständig entfernt.

Eine elektrisch leitfähige Verbindung stellt sich im Cermet in der Regel dann ein, wenn der Metallgehalt über der sogenannten Perkolationsschwelle liegt, bei der die Metallpartikel im gesinterten Cermet mindestens punktuell miteinander verbunden sind, so dass eine elektrische Leitung ermöglicht wird. Dazu muss der Metallgehalt erfahrungsgemäß, abhängig von der Materialauswahl, mindestens 25 Vol.-% betragen.

Die keramische Komponente des Cermets ist bevorzugt ausgewählt aus der Gruppe bestehend aus einer Oxidkeramik, einer Silikatkeramik, einer Nichtoxid-Keramik und einer Elementkeramik oder einer Mischung aus mindestens zwei davon.

Die Oxidkeramik ist bevorzugt ausgewählt aus der Gruppe bestehend aus einem Metalloxid, einem Halbmetalloxid oder einer Mischung davon. Das Metall des Metalloxids kann ausgewählt sein aus der Gruppe bestehend aus Aluminium, Beryllium, Barium, Calcium, Magnesium, Natrium, Kalium, Eisen, Zirkonium, Titan oder einer Mischung von mindestens zwei davon. Das Metalloxid ist bevorzugt ausgewählt aus der Gruppe bestehend aus Aluminiumoxid (Al₂O₃), Magnesiumoxid (MgO), Zirkoniumoxid (ZrO₂), Yttriumoxid (Y₂O₃), Aluminiumtitanat (Al₂TiO₅), einer Piezokeramik wie Blei-Zirkonat (PbZrO₃), Blei-Titanat (PbTiO₃) sowie Blei-Zirkonat-Titanat (PZT) oder einer Mischung von mindestens zwei hiervon. Das Halbmetall des Halbmetalloxids ist bevorzugt ausgewählt aus der Gruppe bestehend aus Bor, Silicium, Arsen, Tellur oder einer Mischung von mindestens zwei davon. Eine weitere bevorzugte Oxidkeramik beinhaltet eines ausgewählt aus der Gruppe bestehend aus Zirkoniumoxid verstärktes Aluminiumoxid (ZTA-Zirconia Toughened Alumina - Al₂O₃/ZrO₂), Yttrium verstärktes Zirkoniumoxid (Y-TZP), Barium(Zr, Ti)oxid, Barium(Ce, Ti)oxid oder eine Kombination aus mindestens zwei davon.

Die Silikatkeramik ist bevorzugt ausgewählt aus der Gruppe bestehend aus einem Steatit (Mg₃[Si₄O₁₀(OH)₂]), Cordierit (Mg, Fe²⁺)₂(Al₂Si)[Al₂Si₄O₁₈]), Mullit (Al₂Al₂₊₂ₓSi₂₋₂ₓO₁₀₋ₓ mit x = Sauerstoffleerstellen pro Elementarzelle), Feldspat (Ba,Ca,Na,K,NH₄)(Al,B,Si)₄O₈) oder einer Mischung aus mindestens zwei davon.

Die Nichtoxid-Keramik kann ausgewählt sein aus der Gruppe bestehend aus einem Carbid, einem Nitrid oder einer Mischung daraus. Das Carbid kann ausgewählt sein aus der Gruppe bestehend aus Siliciumcarbid (SiC), Borcarbid (B₄C), Titancarbid (TiC), Wolframcarbid, Zementit (Fe3C). Das Nitrid kann ausgewählt sein aus der Gruppe bestehend aus Siliciumnitrid (Si₃N₄), Aluminiumnitrid (AIN), Titannitrid (TiN), Siliciumaluminiumoxinitrid (SIALON) oder einer Mischung aus mindestens zwei davon. Eine weitere bevorzugte Nichtoxid-Keramik ist Natrium-Kalium-Niobat.

Die Elementkeramik ist bevorzugt Kohlenstoff, besonders bevorzugt Diamant. Zum Herstellen eines Cermets, welches als keramische Komponente eine Elementkeramik beinhaltet wird bevorzugt ein Diamantpulver mit einem Metallpulver gemischt. Ein solcher Cermet mit einer Elementkeramik als keramischer Komponente zeichnet sich durch eine besonders bevorzugte Kombination aus hoher Härte und hoher spezifischer Wärmeleitfähigkeit aus.

Für die metallische Komponente des Cermets kommen alle dem Fachmann geläufigen Metalle in Betracht, die eine gute Verträglichkeit mit eukaryotischem Gewebe aufweisen. Ein bevorzugtes Metall ist vorzugsweise ausgewählt aus der Gruppe bestehend aus Platin, Iridium, Niob, Palladium, Eisen, Edelstahl, Kobald-Chrom-Legierung, Molybdän, Tantal, Wolfram, Titan, Kobalt und Zirkonium oder eine Kombination von mindestens zwei davon. Dabei ist eine bevorzugte Kombination eine Legierung. Ein bevorzugter Edelstahl ist ein Edelstahl 316L. Ein bevorzugtes Metall ist biokompatibel. Eine bevorzugte Legierung ist biokompatibel. Ein bevorzugtes biokompatibles Metall ist eines ausgewählt aus der Gruppe bestehend aus Palladium, Rhodium, Ruthenium, Molybdän, Platin, Iridium, Wolfram, Gold, Titan, Niob und Tantal oder eine Kombination aus mindestens zwei davon.

Der Grundkörper und das wenigstens eine Leitungselement sind durch eine stoffschlüssige gesinterte Verbindung miteinander verbunden. Stoffschlüssig hat die Bedeutung, dass die beiden zu verbindenden Teile nach dem Verbinden eine Einheit bilden und die Verbindung selbst eine Festigkeit aufweist, die mindestens der einer der beiden Teile entspricht. Dies kann zur Folge haben, dass die verbundenen Teile bei mechanischer- oder Druckbelastung nicht an der Verbindungsstelle auseinander brechen, sondern an einer anderen Stelle der beiden verbundenen Teile. Hierdurch kann gewährleistet werden, dass die Verbindung genauso oder weniger porös oder gas- oder feuchtigkeitsdurchlässig ist wie die zu verbindenden Teile selbst.

Unter einem Sintern, einem Sinterprozess oder co-Sintern wird im Rahmen der vorliegenden Erfindung allgemein ein Verfahren zur Herstellung von Werkstoffen oder Werkstücken verstanden, bei welchem pulverförmige, insbesondere eines ausgewählt aus der Gruppe bestehend aus feinkörnige Stoffe, keramische Stoffe und metallische Stoffe oder eine Kombination aus mindestens zwei davon erhitzt und dadurch verbunden werden. Dieser Prozess kann ohne äußeren Druck auf den zu erhitzenden Stoff erfolgen oder kann insbesondere unter erhöhtem Druck auf den zu erhitzenden Stoff erfolgen, beispielsweise unter einem Druck von mindestens 2 bar, vorzugsweise höheren Drücken, beispielsweise Drücken von mindestens 10 bar, insbesondere mindestens 100 bar oder sogar mindestens 1000 bar. Der Prozess kann insbesondere vollständig oder teilweise bei Temperaturen unterhalb der Schmelztemperatur der pulverförmigen Werkstoffe erfolgen, beispielsweise bei Temperaturen von 700°C bis 1400 °C. Der Prozess kann insbesondere vollständig oder teilweise in einem Werkzeug oder einer Form oder beides durchgeführt werden, so dass mit dem Sinterprozesses eine Formgebung verbunden werden kann. Neben den pulverförmigen Werkstoffen kann ein Ausgangsmaterial für den Sinterprozess weitere Werkstoffe umfassen, beispielsweise einen oder mehrere Binder oder ein oder mehrere Lösungsmittel oder beides. Der Sinterprozess kann in einem Schritt oder auch in mehreren Schritten erfolgen, wobei dem Sinterprozess beispielsweise weitere Schritte vorgelagert sein können, beispielsweise ein oder mehrere Formgebungsschritte oder ein oder mehrere Entbinderungsschritte oder beides. Das Sintern bzw. der Sinterprozess entspricht somit einem Brennprozess. Der Sinterprozess, insbesondere für ein Cermet, kann vergleichbar zu einem üblicherweise für homogene Pulver verwendeten Sinterprozess ablaufen. Beispielsweise kann unter hoher Temperatur und ggf. hohem Druck das Material beim Sintervorgang verdichtet werden, so dass das Cermet nahezu dicht ist, oder eine höchstens geschlossene Porosität aufweist. Cermets zeichnen sich in der Regel durch eine besonders hohe Härte und Verschleißfestigkeit aus.

Eine bevorzugte Ausführungsform der elektrischen Durchführung ist dadurch gekennzeichnet, dass das Kontaktelement den leitfähigen Bereich vollständig überlagert.

Weiter bevorzugt ist eine Ausführungsform der elektrischen Durchführung die dadurch gekennzeichnet ist, dass der Betrag der Fläche des schichtartigen Kontaktelements nicht mehr als 25 % größer ist als der Betrag der Fläche des überlagerten leitfähigen Bereichs, bevorzugt nicht mehr als 10 % größer ist, besonders bevorzugt nicht mehr als 5 % größer ist. Weiter bevorzugt ist, dass beide Flächen im Wesentlichen kongruent oder kongruent zueinander sind. Speziell bevorzugt ist der Betrag der Fläche des Kontaktelements nicht mehr als 1 % größer als der Betrag der Fläche des überlagerten leitfähigen Bereichs des Leitungselements.

Unter der Fläche des elektrisch leitfähigen Kontaktelements wird diejenige Fläche verstanden, die sich aus der Projektion derjenigen Fläche ergibt, die bei einer senkrechten Draufsicht auf die elektrische Durchführung erkennbar ist. Im Idealfall entspricht die Fläche des Kontaktelements der Fläche des elektrisch leitfähigen Bereichs des Leitungselements innerhalb der Oberfläche des Grundkörpers und beide Flächen sind vollständig kongruent. In diesem Fall würde das Kontaktelement den leitfähigen Bereich des Leitungselements innerhalb der Oberfläche des Grundkörpers vollständig randlos überdecken. Wie bereits oben ausgeführt beginnt mit fortschreitender elektrochemischer Abscheidung ein Wachstum des schichtartigen Kontaktelements in radialer oder lateraler Richtung. Dadurch ist der Betrag der Fläche des Kontaktelements in der Regel etwas größer ist als die Fläche des leitfähigen Bereichs des Leitungselements. Trotzdem kann davon ausgegangen werden, dass beide Flächen in Folge der elektrochemischen Abscheidung weitgehend kongruent, also annähernd deckungsgleich sind.

Dies hat eine Reihe von Vorteilen. Durch die Deckungsgleichheit wird bei der Verwendung von Edelmetall für das Kontaktelement wertvolles Material eingespart, da nur jene Flächen beschichtet sind, die wirklich zur Kontaktierung mit einem Metalldraht benötigt werden.

Durch die Deckungsgleichheit wird auch ein anschließender, automatisierter Fügeprozess zur Anbringung eines Metalldrahtes an das Kontaktelement erleichtert. Ist das Kontaktelement bezüglich des Betrages seiner Fläche merklich größer als der elektrisch leitfähige Bereich des Leitungselements, könnte eine optische Erkennung nicht genau identifizieren, wo exakt sich das Leitungselement unterhalb des Kontaktelements befindet. Ist das Kontaktelement bezüglich des Betrages seiner Fläche merklich kleiner als der elektrisch leitfähige Bereich des Leitungselements, wäre insbesondere bei Leitungselementen mit kleinen Durchmessern das Anbringen des Drahtes an das Kontaktelement schwierig. Es könnte dann nur mit sehr feinen Drähten gearbeitet werden. In diesem Fall ist es somit besonders wichtig, die gesamte durch das Leitungselement zur Verfügung gestellte Fläche auszunutzen. Dadurch können Drähte mit größeren Durchmessern verwendet werden und der Widerstand des Systems kann minimiert werden. Insbesondere trifft dies auf den Übergangswiderstand vom Cermet-Leitungselement zum Kontaktelement zu.

Eine bevorzugte Ausführungsform der elektrischen Durchführung ist dadurch gekennzeichnet, dass das Cermet einen Metall-Anteil von wenigstens 25 Vol.-% aufweist, bevorzugt einen Metall-Anteil von 30 Vol.-% bis 90 Vol.-% beinhaltet, besonders bevorzugt einen Metall-Anteil von 35 Vol.-% bis 60 Vol.-% beinhaltet, weiter bevorzugt einen Metall-Anteil von 40 Vol.-% bis 50 Vol.-% beinhaltet.

Der Volumenanteil des Metalls ist definiert als der Quotient des Volumenanteils der Metallpartikel durch die Summe aus dem Volumenanteil der Metallpartikel und dem Volumenanteil der Keramikpartikel.

Das Cermet muss über einen Mindestmenge an Metallpartikeln verfügen, damit die Perkolationsschwelle erreicht und überschritten wird. Überraschenderweise wurde festgestellt dass ein Metall-Anteil von 25 Vol.-% ausreichend ist, um ein niedrigporöses und damit dichtes und festhaftendes erfindungsgemäßes Kontaktelement zu erhalten. Trotz des niedrigen Gehalts an Metallpartikeln scheint eine ausreichend hohe Anzahl von Keimstellen an der Oberfläche vorhanden zu sein, so dass bei der elektrochemischen Abscheidung ein entsprechend dichtes und fest haftendes Kontaktelement erhalten wird. Eine ausreichende Hermetizität der Verbindung zwischen dem Leitungselement und dem Grundkörper ist oberhalb von 90 Vol.-% Metall-Gehalt nicht mehr zwangsläufig gewährleistet, da die stoffschlüssige Verbindung zwischen dem Leitungselement und dem Grundkörper durch das zusammensintern oder co-sintern der Keramikpartikel im Cermet und im Grundkörper erreicht wird.

Eine bevorzugte Ausführungsform der elektrischen Durchführung ist dadurch gekennzeichnet, dass das Kontaktelement eine mittlere Schichtdicke zwischen 1 µm und 50 µm aufweist, bevorzugt eine mittlere Schichtdicke zwischen 5 µm und 35 µm aufweist, besonders bevorzugt eine mittlere Schichtdicke zwischen 10 µm und 20 µm aufweist, und weiter bevorzugt eine Schichtdicke von 15 µm aufweist.

Um zugfeste Verbindungen zwischen einem Metalldraht und dem Kontaktelement herzustellen, sind die genannten Schichtdicken bevorzugt. Dabei sollte eine Schichtdicke von 1 µm nicht unterschritten werden, damit das schichtartige Kontaktelemente beispielsweise beim Laserschweißen nicht bereichsweise vollständig aufschmilzt. Dies führt dazu, dass bereichsweise eine direkte Verbindung zwischen dem Metalldraht und dem Cermet-Leitungselement erzeugt wird, die in bestimmten Fällen nicht über eine ausreichende Zugfestigkeit verfügt. Zu große Schichtdicken resultieren in langen Beschichtungszeiten und können dadurch impraktikabel und unwirtschaftlich werden.

Eine bevorzugte Ausführungsform der elektrischen Durchführung ist dadurch gekennzeichnet, dass die poröse Struktur des Kontaktelements zu wenigstens 70 Vol.-% eine geschlossene Porosität aufweist, bevorzugt zu wenigstens 90 Vol.-% eine geschlossene Porosität aufweist und besonders bevorzugt vollständig geschlossen porös ist. Die in diesem Zusammenhang gemachten Angaben in Vol.-% stellen den Anteil des Volumens vom gesamten Porenvolumen dar.

Durch die bei der elektrochemischen Abscheidung verwendeten Parameter, wie beispielsweise der Stromdichte und der Konzentration des Elektrolyten, ist einstellbar, ob das Kontaktelement nach der Abscheidung eine geschlossene oder offene Porosität oder Mischformen davon aufweist. Geschlossene Porosität bedeutet, dass die Poren nicht miteinander in Verbindung stehen. Offene Porosität bedeutet, dass die Poren untereinander in Verbindung stehen. Es wurde festgestellt, dass eine geschlossen poröse Form zu mechanisch stabileren Verbindungen zwischen der drahtähnlichen Struktur und dem Kontaktelement führt.

Dabei ist eine weitere bevorzugte Ausführungsform der elektrischen Durchführung dadurch gekennzeichnet, dass das Kontaktelement eine Porosität von 0,1 bis 10 Vol.-% aufweist, bevorzugt von 0,2 bis 2 Vol.-% aufweist, besonders bevorzugt von 0,2 bis 0,5 Vol.% aufweist.

Durch elektrochemische Abscheidung erzeugte Schichten des Kontaktelements haften besonders gut an dem überlagerten Cermet-Leitungselement, wenn die Porosität des Kontaktelements in einem der als bevorzugt genannten Bereich liegt.

Eine bevorzugte Ausführungsform der elektrischen Durchführung ist dadurch gekennzeichnet, dass das Kontaktelement einen Porositätsgradienten aufweist.

Durch eine entsprechende Auswahl der Parameter bei der elektrochemischen Abscheidung kann die elektrochemisch erzeugte Schicht des Kontaktelements mit einem Porositätsgradienten versehen werden. Dies ist insbesondere durch eine Variation der Stromstärke bei der galvanischen Beschichtung leicht möglich. Entsprechend werden bei niedrigen Stromdichten Kontaktelemente mit niedrigerer Porosität erhalten. Bei hohen Stromdichten werden Kontaktelemente mit höherer Porosität erhalten. Tendenziell steigt die Porosität somit mit der bei der Abscheidung angelegten Stromdichte an.

Dabei ist eine weitere bevorzugte Ausführungsform der elektrischen Durchführung dadurch gekennzeichnet, dass die mittlere Porosität des an das Leitungselement angrenzenden Bereichs am niedrigsten ist und die mittlere Porosität mit zunehmendem Abstand von dem an das Leitungselement angrenzenden Bereich ansteigt.

Eine bevorzugte Ausführungsform der elektrischen Durchführung ist dadurch gekennzeichnet, dass die gemittelte Rautiefe Rz des schichtartigen Kontaktelements zwischen 0,2 µm und 20 µm beträgt, bevorzugt zwischen 0,5 µm und 12 µm beträgt, besonders bevorzugt zwischen 1 µm und 6 µm beträgt.

Kleine Rautiefen, d.h. eine hohe Güte der Oberflächenqualität, führen zu höheren Zugfestigkeiten eines an ein Kontaktelement angeschweißten Metalldrahts. Es wurde überraschend gefunden, dass niedrige Rautiefen bei der elektrochemischen Beschichtung insbesondere dann erhalten werden, wenn das Substrat (Grundkörper und elektrisch leitfähiger Bereich des Leitungselements innerhalb des Grundkörpers) durch glatt Schleifen und Polieren vorbehandelt wird.

Eine bevorzugte Ausführungsform der elektrischen Durchführung ist dadurch gekennzeichnet, dass das für das Cermet des Leitungselements eingesetzte Metall-Pulver eine mittlere Partikelgröße von 0,5 µm bis 5 µm aufweist, bevorzugt eine mittlere Partikelgröße von 0,5 µm bis 3 µm aufweist, besonders bevorzugt eine mittlere Partikelgröße von 0,7 µm bis 2 µm aufweist und weiter bevorzugt eine mittlere Partikelgröße von 0,9 µm bis 1,9 µm aufweist.

Unter "mittlerer Partikelgröße" wird dabei der d₅₀-Wert der Partikelgrößenverteilung, auch 50%-Quantil oder Medianwert verstanden. Metall-Partikel mit kleiner mittlerer Partikelgröße ermöglichen eine große Anzahl von Keimstellen bei der elektrochemischen Beschichtung, so dass unter diesen Umständen fest haftende und dichte Schichten mit niedriger Porosität erhalten werden. Weiter bevorzugt sollte die Breite der Partikelgrößenverteilung nicht zu groß sein, das heißt es dürfen keine zu großen Partikel in der Metallpulver-Fraktion vorhanden sein. Bevorzugt hat die ausgewählte Fraktion des Metall-Pulvers eine Breite von insgesamt nicht mehr als 10 µm, bevorzugt nicht mehr als 8 µm und besonders bevorzugt von nicht mehr als 5 µm.

Dabei ist eine weitere bevorzugte Ausführungsform der elektrischen Durchführung dadurch gekennzeichnet, dass das für das Cermet eingesetzte Keramik-Pulver eine mittlere Partikelgröße von 0,1 µm bis 10 µm aufweist, bevorzugt von 0,5 µm bis 4 µm aufweist, besonders bevorzugt von 1 µm bis 2,5 µm aufweist und weiter bevorzugt von 1,1 µm bis 1,8 µm aufweist.

Auch das für das Cermet des Leitungselements eingesetzte Keramik-Pulver darf keine zu großen Partikel in der ausgewählten Keramikpartikel-Fraktion aufweisen. Sind zu große Partikel im Keramikpulver vorhanden, werden die mittleren Abstände zwischen den Metall-Partikeln zu groß. Auch wenn in diesem Fall genügend viele Keimstellen am Metall gebildet werden, verfügt die wachsende Schicht aufgrund der großen mittleren Abstände zwischen den Metallpartikeln über eine wenigstens teilweise zu große Porosität oder zumindest ungünstige Porositätsverteilung. Weiter bevorzugt sollte die Breite der Partikelgrößenverteilung des Keramikpulvers nicht zu groß sein, das heißt es dürfen keine zu großen Partikel in der Keramikpulver-Fraktion vorhanden sein. Bevorzugt hat die ausgewählte Fraktion des Keramikpulvers eine Breite von insgesamt nicht mehr als 20 µm, bevorzugt nicht mehr als 15 µm und besonders bevorzugt von nicht mehr als 10 µm.

Eine bevorzugte Ausführungsform der elektrischen Durchführung ist dadurch gekennzeichnet, dass das Leitungselement wenigstens ein Metall ausgewählt aus der Gruppe bestehend aus Platin, Iridium, Niob, Molybdän, Tantal, Wolfram, Titan, Kobalt, Chrom und Zirkonium beinhaltet oder das Leitungselement eine Legierung aus wenigstens zwei der vorgenannten Metalle beinhaltet.

Eine bevorzugte Ausführungsform der elektrischen Durchführung ist dadurch gekennzeichnet, dass das Kontaktelement wenigstens ein Metall ausgewählt aus der Gruppe bestehend aus Gold, Silber, Palladium, Platin, Kupfer, Chrom, Nickel und Eisen oder eine Legierung aus wenigstens zwei der vorgenannten Metalle beinhaltet.

Das Kontaktelement kann aus jedem Material erzeugt sein, das dem Fachmann bekannt ist, um eine elektrisch leitfähige Verbindung zwischen zwei Körpern herzustellen. Aus diesem Grund ist das Kontaktelement bevorzugt aus einem Metall. Besonders bevorzugt sind dabei Metalle, Mischungen oder Legierungen, die mindestens zum Teil ausgewählt sind aus der Gruppe, bestehend aus Gold, Silber, Palladium, Platin, Kupfer, Chrom, Nickel und Eisen.

Bevorzugt sind die für das Cermet des Leitungselements und die Verbindungsschicht ausgewählten Metalle, Mischungen davon und Legierungen biokompatibel, weshalb sie zum Einsatz in implantierbaren Vorrichtungen gut geeignet sind. Ein bevorzugtes biokompatibles Metall ist ausgewählt aus der Gruppe bestehend aus biotolerant, bioinert und bioaktiv oder eine Kombination aus mindestens zwei davon. Biokompatibiltät kann beispielsweise mit der Norm ISO 10933-4:2002 bestimmt werden.

Betrachtungen für die Biokompatibilität gelten in bevorzugten Ausführungsformen der elektrischen Durchführung auch für die Keramik-Komponenten des Cermets, des Grundkörpers und gegebenenfalls weitere Bestandteile der elektrischen Durchführung.

Eine bevorzugte Ausführungsform der elektrischen Durchführung ist dadurch gekennzeichnet, dass der elektrisch isolierende Grundkörper eine Keramik aufweist.

Der Grundkörper kann insbesondere ganz oder teilweise aus einem oder mehreren sinterfähigen Materialien hergestellt sein, insbesondere aus einem oder mehreren sinterfähigen Materialien auf Keramikbasis.

Eine bevorzugte Ausführungsform der elektrischen Durchführung ist dadurch gekennzeichnet, dass der elektrisch isolierende Grundkörper wenigstens eine Keramik ausgewählt aus der Gruppe Aluminiumoxid (Al₂O₃), Zirkoniumdioxid (ZrO₂), Aluminiumoxid verstärktes Zirkoniumoxid (ZTA), Zirkoniumoxid verstärktes Aluminiumoxid (ZTA - Zirconia Toughened Aluminum - Al₂O₃/ZrO₂), Yttrium verstärktes Zirkoniumoxid (Y-TZP), Aluminiumnitrid (AIN), Magnesiumoxid (MgO), Piezokeramik, Barium(Zr, Ti)oxid, Barium(CE, Ti)oxid, oder Natrium-Kalium-Niobat.

Der Grundkörper kann auch aus einer der oben genannten Keramiken hergestellt sein, die für die Herstellung des Cermets Verwendung finden.

Eine bevorzugte Ausführungsform der elektrischen Durchführung ist dadurch gekennzeichnet, dass der Grundkörper und der Cermet die gleiche Keramik aufweisen, bevorzugt der Grundkörper und der Cermet ausschließlich die gleiche Keramik, das heißt keine weitere Keramik aufweisen. Besonders wird dabei Aluminiumoxid bevorzugt.

Eine bevorzugte Ausführungsform der elektrischen Durchführung ist dadurch gekennzeichnet, dass der elektrisch isolierende Grundkörper aus mehreren gesinterten Keramikschichten gebildet ist.

Dabei wird zur Herstellung der Durchführung zunächst eine Grünkörperfolie bereitgestellt und beispielsweise durch Stanzen mit Löchern versehen. Die Löcher werden mit einer geeigneten Cermet-Paste gefüllt. Die Cermet-Paste beinhaltet in diesem Stadium der Herstellung mindestens eine Mischung aus Metall-Pulver, Keramik-Pulver und einem organischen Vehikel. Mehrere der so befüllten Grünkörperfolien werden anschließend laminiert, so dass die Cermetbefüllten Löcher übereinander angeordnet sind. Diese bilden später das Leitungselement. Es werden so viele befüllte Grünkörperfolien laminiert, dass die gewünschte Dicke der elektrischen Durchführung beziehungsweise Länge des Leitungselements erreicht wird. Beim anschließenden Brennen wird zunächst das organische Vehikel entfernt, beim anschließenden Übergang zu höheren Temperaturen werden das Cermet und der keramische Grundkörper co-gesintert. Dabei entsteht insbesondere eine hermetisch dichte stoffschlüssige Verbindung zwischen der Keramikkomponente des Cermets und der umgebenden Keramik des Grundkörpers.

Eine bevorzugte Ausführungsform der elektrischen Durchführung ist dadurch gekennzeichnet, dass die elektrische Durchführung eine Helium-Leckrate von weniger als 1 ^{∗} 10⁻⁷ atm^{∗}cm³/sec aufweist. Eine elektrische Durchführung mit einer solchen Helium-Leckrate kann als "hermetisch dicht" bezeichnet werden.

Der Begriff "hermetisch dicht" verdeutlicht, das bei einem bestimmungsgemäßen Gebrauch innerhalb der üblichen Zeiträume (beispielsweise 5-10 Jahre) Feuchtigkeit und/oder Gase nicht oder nur minimal durch das hermetisch dichte Element hindurch dringen können. Eine physikalische Größe, die beispielsweise eine Permeation von Gasen und/oder Feuchtigkeit durch eine Vorrichtung, z. B. durch die elektrische Durchführung, beschreiben kann, ist die sogenannte Leckrate, welche beispielsweise durch Lecktests bestimmt werden kann. Entsprechende Lecktests können beispielsweise mit Heliumlecktestern durchgeführt werden und sind im Standard Mil-STD-883G Method 1014 spezifiziert. Die maximal zulässige Helium-Leckrate wird dabei abhängig vom internen Volumen der zu prüfenden Vorrichtung festgelegt. Nach den in MIL-STD-883G, Method 1014, in Absatz 3.1 spezifizierten Methoden, und unter Berücksichtigung der in der Anwendung der vorliegenden Erfindung vorkommenden Volumina und Kavitäten der zu prüfenden Vorrichtungen, können diese maximal zulässigen Helium-Leckraten beispielsweise von 1 x 10⁻⁸ atm·cm³/sec bis 1 x 10⁻⁷ atm·cm³/sec betragen. Im Rahmen der Erfindung kann der Begriff "hermetisch dicht" insbesondere bedeuten, dass die zu prüfende Vorrichtung, insbesondere die elektrische Durchführung, eine Helium-Leckrate von weniger als 1 x 10⁻⁷ atm·cm³/sec aufweist. In einer vorteilhaften Ausführung kann die Helium-Leckrate weniger als 1 x 10⁻⁸ atm·cm³/sec, insbesondere weniger als 1 x 10⁻⁹ atm·cm³/sec betragen. Zum Zweck der Standardisierung können die genannten Helium-Leckraten auch in die äquivalente Standard-Luft-Leckrate konvertiert werden. Die Definition für die äquivalente Standardluft-Leckrate (Equivalent Standard Air Leak Rate) und die Umrechnung sind im Standard ISO 3530 angegeben.

Einen Beitrag zur Erfüllung mindestens einer der erfindungsgemäßen Aufgaben leistet ferner ein Verfahren, beinhaltend als Verfahrensschritte:
a) Bereitstellen einer elektrischen Durchführung, wobei die Durchführung
   i. einen elektrisch isolierenden Grundkörper und
      ein elektrisches Leitungselement aufweist, wobei das Leitungselement ein Cermet beinhaltet,
   ii. der Grundkörper und das Leitungselement durch eine stoffschlüssige gesinterte Verbindung verbunden sind, so dass das Leitungselement hermetisch gegen den Grundkörper abgedichtet ist,
   iii. sich das Leitungselement von einer ersten Oberfläche des Grundkörpers durch den Grundkörper hindurch zu einer zweiten Oberfläche des Grundkörpers erstreckt,
   iv. das Leitungselement einen ersten elektrisch leitfähigen Bereich innerhalb der ersten Oberfläche des Grundkörpers und einen zweiten elektrisch leitfähigen Bereich innerhalb der zweiten Oberfläche des Grundkörpers aufweist,
b) Aufbringen einer elektrisch leitenden Elektrodenschicht auf die erste Oberfläche des Grundkörpers, so dass eine leitfähige Verbindung zwischen dem ersten leitfähigen Bereich des Leitungselements und der Elektrodenschicht gebildet wird,
c) Einbringen der Durchführung in eine Metall-Elektrolyt-Lösung und Bilden eines metallischen Kontaktelements im zweiten elektrisch leitfähigen Bereich des Leitungselements mittels elektrochemischer Abscheidung durch Reduktion von Kationen der Metall-Elektrolyt-Lösung,
d) Entfernen der Elektrodenschicht.

Die Elektrodenschicht in Schritt b) dient der Herstellung einer zuverlässigen elektrischen Verbindung zwischen dem Cermet-Leitungselement und der Kathode einer Gleichstromquelle. Das heißt die Kathode der Gleichstromquelle wird über die Elektrodenschicht mit der elektrischen Durchführung verbunden. Dabei muss sichergestellt werden, dass eine Kontaktierung zwischen dem Cermet-Leitungselement und der Elektrodenschicht gewährleistet ist. Da das Cermet trotz relativ geringen Metall-Anteils im Verbund über zahlreiche durchgehende elektrische Pfade verfügt, kommt es bei Anlegen von Gleichstrom und Schalten der Elektrodenschicht als Kathode zu einem Elektronen-Überschuss sowohl im Bereich der Elektrodenschicht als auch am zweiten elektrisch leitfähigen Bereich, also an der frei liegenden Fläche des Cermet-Leitungselements. Dadurch werden in diesen Bereichen Metall-Kationen zu elementarem Metall elektrochemisch abgeschieden. Als Voraussetzung dafür muss die Durchführung insgesamt in eine Metall-Elektrolyt-Lösung eingetaucht werden, so lange eine elektrochemische Metall-Abscheidung erfolgen soll. Gleichzeitig taucht in die Elektrolyt-Lösung eine vorzugsweise inerte Elektrode ein, die als Anode dient. Die Elektrodenschicht wird nach Bildung des metallischen Kontaktelements wieder entfernt, um die erste Oberfläche des Grundkörpers und den ersten leitfähigen Bereich des Leitungselements oder der Leitungselemente freizulegen.

Optional kann anschließend eine weitere Elektrodenschicht gemäß Schritt b) auf die zweite Oberfläche des Grundkörpers, welche bereits mit einem oder mehrere Kontaktelementen im Bereich des zweiten leitfähigen Bereichs des Leitungselements oder der Leitungselemente versehen wurde, aufgebracht werden. Anschließend werden die Schritte c) und d) wiederholt, um auch im ersten leitfähigen Bereich des Leitungselements oder der Leitungselemente metallische Kontaktelemente elektrochemisch zu erzeugen.

In einer bevorzugten Ausführungsform ist das Verfahren dadurch gekennzeichnet, dass in einem weiteren Verfahrensschritt b-1) nach Schritt b) eine die Elektrodenschicht überlagernde Isolierschicht aufgebracht wird, so dass keine elektrochemische Abscheidung eines Metalls im Bereich der Elektrodenschicht stattfindet.

Insbesondere bei der elektrochemischen Abscheidung von wertvollen Edelmetallen ist es unerwünscht, dass die Abscheidung des Metalls aus der Elektrolyt-Lösung nicht nur im Bereich des Leitungselements, sondern auch an der Elektrodenschicht erfolgt. In diesem Fall wird die Elektrodenschicht wenigstens teilweise mit einer die Elektrodenschicht überlagernden Isolierschicht versehen. Dadurch kann in den überlagernden Bereichen keine Metall-Abscheidung an der Elektrodenschicht erfolgen. Die Isolierschicht kann auch jedem für den Fachmann geeigneten Material bestehen, beispielsweise aus einem Kunststoff oder aus einer Keramik.

In einer bevorzugten Ausführungsform ist das Verfahren dadurch gekennzeichnet, dass in einem weiteren Verfahrensschritt b-2) vor Durchführung von Schritt c) die elektrische Durchführung in eine alkalische Lösung eingetaucht wird, wobei die Elektrodenschicht mit einer Gleichstromquelle verbunden und als Kathode geschaltet wird, um die Oberflächen der elektrischen Durchführung zu reinigen.

Es hat sich als vorteilhaft erwiesen, wenn die Oberflächen der elektrischen Durchführung, insbesondere die Oberflächen der leitfähigen Bereiche des Cermet-Leitungselements vor der eigentlichen elektrochemischen Abscheidung gereinigt werden. Dafür wird das Leitungselement mit der Gleichstromquelle verbunden, als Kathode geschaltet und in eine alkalische Lösung eingetaucht. Als Anode kann beispielsweise ein platiniertes Titan-Streckmetall dienen. Beim Anlegen von Gleichstrom beobachtet man eine starke Gasentwicklung im Bereich der Kathode, was auf die Entwicklung von molekularem Wasserstoff durch Reduktion von Wassergebundenen Protonen (Oxonium-Ionen) zurückzuführen ist. Die Gasblasen reißen lose Partikel, anhaftende Fette, Öle und andere Verunreinigungen mit, wodurch diese von der Oberfläche entfernt werden. Darüber hinaus löst die alkalische Lösung dünne Keramik-Filme von der Cermet-Oberfläche ab, wodurch diese Oberflächen der elektrochemischen Abscheidung besser zugänglich werden.

In einer bevorzugten Ausführungsform ist das Verfahren dadurch gekennzeichnet, dass die alkalische Lösung Natriumhydroxid und/oder Kaliumhydroxid beinhaltet und optional einen Zusatz eines Cyanid-Salzes beinhaltet.

Die Reinigung der Oberflächen ist durch die Verwendung von starken alkalischen Agentien wie Natrium- oder Kaliumhydroxid besonders effektiv. Andere starke Basen aus der Gruppe Alkalihydroxide, Erdalkalihydroxide, Phosphate, Carbonate und Sulfide können ebenfalls verwendet werden.

Mit Hilfe von Cyaniden können darüber hinaus metallische Verunreinigungen leicht entfernt werden, da Cyanide über gute Komplexbildungseigenschaften verfügen, das heißt hohe Komplexbildungskonstanten mit vielen Metall-Ionen bilden.

In einer bevorzugten Ausführungsform ist das Verfahren dadurch gekennzeichnet, dass das Aufbringen der elektrisch leitenden Elektrodenschicht in Schritt b) durch Anpressen eines elektrisch leitfähigen Polymers an die zweite Oberfläche erfolgt.

Leitfähige Polymere zeichnen sich in vielen Fällen durch ein hohes Maß an Flexibilität aus. Dadurch kann eine Elektrodenschicht durch einfaches Anpressen einer leitfähigen PolymerFolie an die Oberfläche der elektrischen Durchführung erzeugt werden. Trotzdem wird eine zuverlässig elektrisch leitende Verbindung zwischen dem Cermet-Leitungselement und der Elektrodenschicht erzeugt, ohne dass eine stoffschlüssige Verbindung zwischen dem Leitungselement und der Elektrodenschicht hergestellt werden muss. Stoffschlüssig verbundenen Elektrodenschichten sind im Nachgang schwieriger zu entfernen. Beispielsweise erfordern durch Drucken, PVD oder Kathodenzerstäubung aufgebrachte Elektrodenschichten eine nachträgliche maschinelle Entfernung.

Als leitfähige Polymere sind alle dem Fachmann bekannten Polymere einsetzbar, bevorzugt sind intrinsisch leitfähige Polymere. Insbesondere bevorzugt sind Polyanilin, Polythiophen, Polythieno-thiophen, Poly-3,4-ethylendioxy-thiophen (PEDOT), Polypyrrol und Copolymere aus den Monomeren dieser Polymere und Polymere oder Copolymere aus den Derivaten dieser Monomeren zu nennen. Besonders bevorzugt findet Poly-3,4-ethylendioxy-thiophen Anwendung. Ja nach Leitfähigkeit können die genannten Polymere auch dotiert vorliegen.

In einer bevorzugten Ausführungsform ist das Verfahren dadurch gekennzeichnet, dass die Elektrodenschicht mehrschichtig ist, wobei der Mehrschicht-Aufbau eine leitfähige Paste und eine die leitfähige Paste überlagernde leitfähige Folie beinhaltet.

Leitfähige Pasten haben den Vorteil, dass diese aufgrund ihrer Viskosität mechanisch einfach zu verarbeiten und gut auf die Oberfläche aufbringbar sind, so dass eine vollständige Kontaktierung des Leitungselements über die gesamte freiliegende Fläche erreicht wird. Auf die Paste kann besonders einfach eine Leitfähige Folie angeklebt werden, die sich für die Verbindung mit der Gleichstromquelle besser eignet als die Paste an sich. Die leitfähige Paste kann ein organisches Vehikel oder Bindemittel enthalten, beispielsweise ein organisches Polymer oder ein Lösungsmittel. Nach dem Aufbringen der Paste wird das Bindemittel gegebenenfalls durch Erhitzen oder durch Anlegen von Vakuum entfernt und dadurch eine noch fester haftende Elektrodenschicht erhalten.

Generell bevorzugt erfolgt die elektrochemische Abscheidung in Schritt c) durch Galvanisieren, wobei die Elektrodenschicht mit einer Gleichstromquelle elektrisch verbunden ist und als Kathode geschaltet wird. Galvanische Verfahren haben grundsätzlich den Vorteil, dass die Geschwindigkeit der Abscheidung und die Dauer einfach gesteuert werden können. Die Geschwindigkeit der Abscheidung, d.h. die Zunahme der Schichtdicke pro Zeiteinheit kann durch die Stromdichte, das heißt durch die Stromstärke pro zu beschichtende Flächeneinheit des Leitungselements oder der Leitungselemente gesteuert werden. Die Zeit der Abscheidung kann einfach durch Zu- und Abschalten des Stromflusses gesteuert werden. Weitere Parameter sind die Konzentration des Elektrolyten in der Elektrolyt-Lösung. Die Elektrolyt-Lösung verarmt mit fortschreitender Zeit der Abscheidung an Metall-Kationen. Daher wird das Absinken der Metall-Kationen-Konzentration mit fortschreitender Abscheidungsdauer vorzugsweise durch Zudosieren höher konzentrierter Elektrolyt-Lösung ausgeglichen.

Beim Galvanisieren taucht neben der elektrischen Durchführung, die als Kathode geschaltet ist, eine inerte Elektrode in die Elektrolyt-Lösung ein. Dabei ist die inerte Elektrode mit einer Gleichstromquelle elektrisch verbunden und wird als Anode geschaltet. Die Anode ist vorzugsweise weitgehend oder sogar vollständig inert. Als Beispiel findet platiniertes Titanstreckmetall Verwendung. Dabei verfügt die Anode vorzugsweise über eine sehr viel größere Oberfläche als das eigentliche Substrat, d.h. die wenigstens eine leitfähige Fläche des Cermet-Leitungselements der elektrischen Durchführung. Dadurch ist die anodische Stromdichte sehr gering. Anodenreaktion ist hauptsächlich die Oxidation von Wasser oder Hydroxid-Ionen zu Sauerstoff. Teilweise werden auch organische Verbindungen, beispielsweise im Elektrolyten verwendete Kornverfeinerer, anodisch zersetzt.

Als inerte Elektrode wird bevorzugt eine Anode ausgewählt aus der Gruppe bestehend aus Gold-Elektrode, vergoldete Kupfer-Elektrode, platinierte Titan-Elektrode und platinierte Niob-Elektrode, wobei platinierte Titan-Elektroden und platinierte Niob-Elektroden bevorzugt sind. Gold ist in Abhängigkeit vom gewählten System unter bestimmten Umständen nicht vollständig inert. Beispielsweise kann sich die Goldschicht der Anode bei Verwendung eines Goldelektrolyten zur Erzeugung eines Kontaktelements aus Gold in den Elektrolyten aus-lösen. Daher sind platinierte Elektroden aufgrund des höheren Standard-Reduktionspotentials von Platin in diesen Fällen bevorzugt.

In einer bevorzugten Ausführungsform ist das Verfahren dadurch gekennzeichnet, dass die Metall-Elektrolyt-Lösung Kationen beinhaltet, ausgewählt aus mindestens einem Element der Gruppe bestehend aus Goldkationen, Silberkationen, Palladiumkationen, Platinkationen, Kupferkationen, Chromkationen, Nickelkationen und Eisenkationen.

Weiter bevorzugt werden Goldkationen und Platinkationen aufgrund der hervorragenden Biokompatibilität dieser Metalle. Insbesondere wird Gold bevorzugt aufgrund der großen Anzahl geeigneter Gold-Verbindungen.

In einer bevorzugten Ausführungsform ist das Verfahren daher dadurch gekennzeichnet, dass die Metall-Elektrolyt-Lösung Kaliumdicyanoaurat(I) und/oder Kaliumtetracyanoaurat(III) beinhaltet.

Alternativ ist das Verfahren dadurch gekennzeichnet, dass die elektrochemische Abscheidung gemäß Schritt c) durch stromlose Metallabscheidung erfolgt. Dabei läuft die Abscheidung durch Auswahl eines geeigneten Redoxsystems spontan ab, ohne dass von außen ein elektrischer Strom angelegt werden muss.

In einer bevorzugten Ausführungsform ist das Verfahren dadurch gekennzeichnet, dass die Stromdichte bei der elektrochemischen Abscheidung gemäß Schritt c) bezogen auf die Fläche des elektrisch leitfähigen Bereichs 0,1 bis 100 A/dm² beträgt, bevorzugt 0,5 bis 30 A/dm² beträgt, besonders bevorzugt 1 bis 15 A/dm² beträgt.

Gegenstand der Erfindung ist weiterhin eine elektrische Durchführung für eine medizinisch implantierbare Vorrichtung, erhältlich durch eine der Ausführungsformen des vorstehend beschriebenen Verfahrens.

Die Ausführungsformen des erfindungsgemäßen Verfahrens finden weiterhin Verwendung zur Herstellung einer erfindungsgemäßen elektrischen Durchführung gemäß einer der vorgenannten Ausführungsformen.

### MESSMETHODEN

Die folgenden Messmethoden wurden im Rahmen der Erfindung benutzt. Sofern nichts anderes angegeben ist wurden die Messungen bei einer Umgebungstemperatur von 25 °C, einem Umgebungsdruck von 100 kPa (0,986 atm) und einer relativen Luftfeuchtigkeit von 50 % durchgeführt.

### Porosität

Zur Messung der Porosität wurden zunächst metallographische Schliffproben durch Einbetten in Epoxidharz, Schleifen mit SiC-Papier mit sukzessive kleinerer Körnung sowie Polieren mit einer Diamantpaste hergestellt. Im Folgenden wurden Aufnahmen der so behandelten Probenoberfläche mit einem Lichtmikroskop und einem Elektronenmikroskop gemacht. Hierbei soll ein möglichst hoher Kontrast zwischen den Poren der Probe und dem Material (Metall und Keramik) erzielt werden. Zur Auswertung der Bilder wurden diese Graustufenbilder mittels Otsu-Methode in binäre Bilder umgewandelt. Das heißt, die Bildpixel wurden mittels eines Schwellenwertes jeweils einer Pore oder dem Probenmaterial zugeordnet. Anschließend wurde die Porosität an Hand der binären Bilder als Quotient aus der Anzahl der Pixel, welche Poren darstellen, und der Gesamtanzahl der Pixel pro Bild ermittelt. Hierbei wurde die Porosität als arithmetischer Mittelwert aus 5 Bildern, jeweils aufgenommen an 5 Schliffproben, bestimmt.

### Bestimmung der Schichtdicke des Kontaktelements

Die Schichtdicke wird mit Hilfe eines lichtoptischen Messmikroskops aus dem Gefüge-Bild eines Querschliffs einer geeigneten Probe als arithmetischem Mittelwert aus 10 Messpunkten ermittelt.

### Gemittelte Rautiefe Rz des Kontaktelements

Die Ermittlung der gemittelten Rautiefe Rz erfolgte berührungslos mit Hilfe eines konfokalen 3D Mikroskops des Herstellers Nanofocus (Typbezeichnung: µsurf explorer) nach der Norm DIN EN ISO 25178-602.

### AUSFÜHRUNGSBEISPIELE

Die Erfindung wird im Folgenden durch ein Ausführungsbeispiel und Zeichnungen genauer dargestellt, wobei das Beispiel und die Zeichnungen keine Einschränkung der Erfindung bedeuten. Die Zeichnungen sind, sofern nicht anders angegeben, nicht maßstabsgetreu.

### Vorbereitung keramischer Grünkörperfolien

Keramische Grünkörperfolien wurden als Keramikvorläufer für den isolierenden Grundkörper eingesetzt. Hierzu wurden 99,7 Gew.-% reine Al₂O₃ - Folien (Keral 99 von Keramische Folien GmbH) der Dicke 400 µm verwendet. Proben der Grünkörperfolien wurden zu 90 mm x 90 mm Quadraten zugschnitten. Etwa kreisrunde Löcher mit einem Durchmesser von 400 µm wurden mit einem mechanischen Stanzeisen (CPC923101 von Groz-Beckert KG) für 400 µm Durchmesser in einer automatisierten Stanze (MP4150 Stanze von Unichem Industries Inc.) in die Folienproben gestanzt. Derart wurden mindestens 4 Folienproben vorbereitet.

### Füllen

Die wie oben vorbereiteten Löcher wurden mit Hilfe einer Schablone von der Christian Koenen GmbH und einem EKRA Microtronic II-Drucker (Modell M2H) mit Cermetpaste gefüllt.

Für die Cermetpaste wurden 60 g eines Platinpulvers mit 24 g eines Al₂O₃-Pulvers mit einem organischen Bindemittel auf Ethylcellulose-Basis gemischt und mit einer 3-Walzenmühle homogenisiert. So erhaltene Pasten hatten Viskositäten in einem Bereich von 250 bis 300 Pa*s (gemessen mit einem Haake Rheostress 6000 Rheometer bei 25 °C) und eine Mahlfeinheit (fineness of grind - FoG) von weniger als 10 µm. Die Rheologie der Pasten war geeignet für den anschließenden Schablonendruck.

Die Dicke der Schablone betrug 100 µm. Die Öffnungen der Schablone hatten die gleichen Abmessungen und Positionen wie die Löcher, welche wie oben beschrieben in die Grünkörperfolie gestanzt worden waren. Die Druckparameter waren 50 N Rakeldruck, Rakelgeschwindigkeit vorwärts 25 mm/s, Rakelgeschwindigkeit rückwärts 25 mm/s und Abriss (snap off) 0,0 mm. Der Rakelkreis wurde so eingestellt, dass Pastenmaterial sowohl bei der Vorwärtsbewegung als auch bei der Rückwärtsbewegung eingebracht wurde.

10 Minuten nach dem Füllen der Proben wurden diese in einen Trockner HHG-2 (von BTU International Inc.) eingebracht und dort für 10 Minuten bei 80 °C getrocknet.

Es wurde eine Füllung von etwa 200 µm Dicke nach dem Drucken (nass) und etwa 150 µm Dicke nach dem Trocknen erreicht. Zum vollständigen Füllen des Lochs der Folie wurden weitere Füllschritte mit der Cermetpaste durchgeführt. Es wurden 1 bis 5 Folienproben durch mehrfaches Ausführen des obigen Füllschrittes mit der Cermetpaste vollständig gefüllt.

### Laminieren der Grünkörperfolien

Es werden 4 Lagen Grünkörperfolie mit wie oben beschrieben gefüllten Löchern mit einem Metallausrichtungswerkzeug gestapelt und in einem Ölbad bei 70 °C mit einem Druck von 350 bar für 10 Minuten isostatisch vepresst (Laminator-CE-1 von Autoclave Engineers), um die gewünschte Bauteildicke von 1,6 mm vor dem Sintern zu erreichen.

### Sintern

Das wie oben erhaltene Laminat von Grünkörperfolien wurde in einem Hochtemperatur-Kammerofen (FHT-175-10-12 der Arnold Schröder Industriehöfen GmbH), geeignet für eine Maximaltemperatur von 1750 °C mit einer Kammergröße von 200 mm x 250 mm x 200 mm gebrannt, um die einzelnen Schichten und Cermet-Füllungen zu sintern. Der Sintervorgang erfolgte unter atmosphärischen Normalbedingungen. Die Temperatur wurde von 25 auf 450 °C bei einer Rate von 30 °C/h erhöht. Dann wurde die Temperatur bei 450 °C für 5 h konstant gehalten, um die organischen Komponenten in dem Grünkörper-Laminat wegzubrennen. Anschließend wurde die Temperatur auf eine Maximaltemperatur in einem Bereich von 1510 bis 1560 °C bei einer Rate von 450 °C/h erhöht und bei diesem Wert für eine Haltedauer in einem Bereich von 1 bis 5 Stunden konstant gehalten. Danach wurde die Temperatur bei einer Kühlrate von 450 °C/h oder der natürlichen Kühlrate, welche langsamer war, auf Raumtemperatur abgesenkt.

Es wurden gesinterte Formkörper mit einem Volumenanteil von 40 Vol.-% bis 45 Vol.-% Platin im Cermet erhalten.

### Nachbehandlung

Nach dem Brennen wurden die Proben abgeschliffen und mit einem Laser auf die gewünschten Abmessungen zugeschnitten.

Die gesinterten Proben wurden auf beiden Seiten auf eine Dicke von 1,0 bis 1,1 mm geschliffen. Aus den geschliffenen Proben wurden einzelne Bereiche mit Hilfe eines Laserschneidverfahrens herausgetrennt. Es wurden Bereiche erhalten, die je Probe 5 Doppelreihen von Cermet-Leitungselementen enthielten.

### Vorbehandlung für die galvanische Beschichtung

Proben wie oben beschrieben wurden zunächst von einer Seite mit einer Elektrodenschicht aus Gold versehen, um für die anschließende galvanische Beschichtung einen ausreichenden Stromfluss zwischen der Gleichstromquelle und dem Cermet-Leitungselement zu gewährleisten. Um die Haftung der Goldschicht auf dem keramischen Grundkörper zu verbessern, wurde eine Zwischenschicht aus Titan aufgebracht. Beide Schichten wurden durch Kathodenzerstäubung ("Sputtern") erzeugt. Die Ziel-Schichtdicken der Titanschicht und der Goldschicht betrugen jeweils zwischen 1 und 2 µm. Das Glimmen erfolgte für 15 Minuten bei einer Spannung von 400 V und einem Argon Volumenstrom von 36 cm³/min mit einem rotierenden Substrathalter. Dabei lag die Probe flach auf der Oberfläche des Substrathalters auf, um die Probe nur einseitig zu beschichten. Die eigentliche Beschichtung durch Kathodenzerstäubung erfolgte bei einem Basisdruck von 5*10⁻⁵ mbar und einem Argon-Volumenstrom von 12 cm³/min.

### Galvanische Beschichtung

Zum Erzeugen der Kontaktelemente auf den freiliegenden Flächen der Cermet-Leitungselemente wurden die Proben über die auf der "Rückseite" der Probe aufgebrachten Goldschicht kontaktiert. Dabei wurde vermieden, dass die Rückseite mit dem verwendeten Gold-Elektrolyten in Kontakt kommt, um eine unnötige Beschichtung der Elektrodenschicht zu vermeiden. Um die Rückseite vor einem Kontakt mit Gold-Elektrolyt zu schützen, wurde die Goldschicht möglichst vollflächig mit mehreren Lagen eines adhäsiven Polymerfilms versehen. Anschließend wurde die Goldschicht mit Hilfe einer Metallklemme mit einer Gleichstromquelle verbunden. Die Klemme diente gleichzeitig der Befestigung der Probe.

Die Probe wurde zunächst zur Entfettung an der Klemme hängend in eine Lösung aus 100 g/l Kaliumhydroxid mit 10 g/l Kaliumcyanid eingebracht. Die Probe wurde als Kathode mit einer Gleichstromquelle (Hersteller: Tandar) verbunden. Als Anode diente ein platiniertes Titan-Streckmetall. Es wurde eine Stromstärke entsprechend einer flächenbezogenen Stromdichte von 5 A/dm² angelegt. Während der kathodischen Entfettung wurde an den freiliegenden leitfähigen Flächen der Kontaktelemente eine starke Gasentwicklung beobachtet. Durch die Gasblasen und die stark alkalische Lösung wurden lose Partikel, anhaftende Fette und andere Verunreinigungen entfernt. Darüber hinaus wurde beobachtet, dass die stark alkalische Lösung dünne Keramik-Filme von den Cermet-Flächen löst.

Nach der kathodischen Entfettung wurde die Probe zur Neutralisation insgesamt dreimal intensiv mit destilliertem Wasser gespült und anschließend in eine Gold-Elektrolytlösung (Enthone, Pura Gold 202 B) auf Basis von Kalium-Gold-Cyanid eingebracht. Die Probe wurde wiederum als Kathode mit der Gleichstromquelle verbunden. Als Anode diente wiederum ein platiniertes Streckmetall. Die Probe wurde im Elektrolyten parallel zur Gegenelektrode (Anode) bewegt. Zusätzlich wurde der Elektrolyt mit Hilfe eines Magnetrührers und Magnetrührstäbchens stark gerührt. Nachdem die Elektrolyt-Lösung mit Hilfe einer Heizplatte auf 80 °C erwärmt wurde, wurde eine Stromdichte von 5 A/dm² angelegt. Nach einer Abscheidungsdauer von 4 Minuten war auf den Leitungselementen Goldschichten mit einer Dicke von 5,3 µm entstanden.

Daraufhin wurde die Probe aus dem Elektrolyt entfernt und intensiv unter fließendem destilliertem Wasser gewaschen. Anschließend wurde mit Ethanol gespült und mit Heißluft bis zur Gewichtskonstanz getrocknet.

Die erhaltenen Kontaktelemente aus Gold wiesen eine mittlere Rautiefe Rz von 1,05 µm und eine Porosität von 0,3 % auf. Damit eigneten sich diese Kontaktelemente zum Anbringen von Drähten mit Hilfe eines Laserschweißverfahrens.

Es zeigen
- Figur 1): eine Querschnittsdarstellung einer erfindungsgemäßen Durchführung;
- Figur 2): eine Querschnittsdarstellung einer weiteren Ausführungsform der erfindungsgemäßen Durchführung;
- Figur 3): eine elektronenmikroskopische Aufnahme einer Draufsicht einer erfindungsgemäßen Durchführung mit Kontaktelementen aus Gold;
- Figur 4): eine implantierbare medizinische Vorrichtung beinhaltend eine erfindungsgemäße Durchführung;
- Figur 5): einen Versuchsaufbau zur Durchführung des erfindungsgemäßen Verfahrens;
- Figur 6): ein Ablaufdiagramm mit den Verfahrensschritten des erfindungsgemäßen Verfahrens.

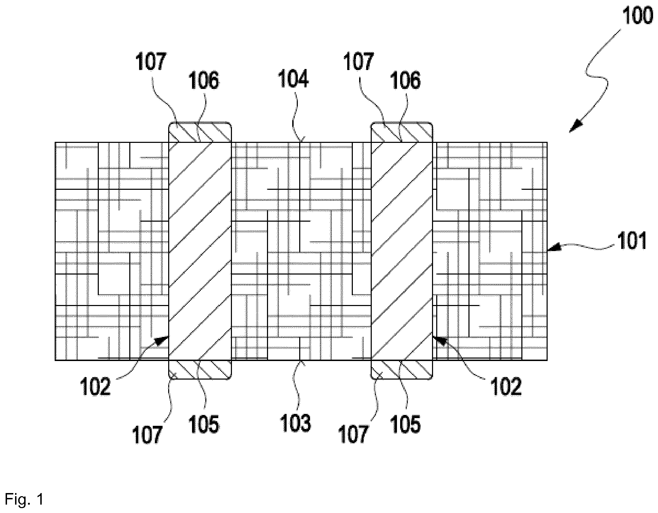

Figur 1 zeigt einen schematischen Querschnitt einer erfindungsgemäßen Durchführung 100. Die Durchführung beinhaltet einen elektrisch isolierenden Grundkörper 101. Der Grundkörper 101 besteht aus einer Keramik, hier aus Aluminiumoxid (Al₂O₃). Der Grundkörper 101 weist eine erste Oberfläche 103 und eine zweite Oberfläche 104 auf. Durch den Grundkörper 101 hindurch erstrecken sich zwei Leitungselemente 102 von der ersten Oberfläche 103 zur zweiten Oberfläche 104 des Grundkörpers 101. Die Oberflächen 103 und 104 sind die ebenen Flächen oder Grundflächen einer Durchführung mit der Form eines Kreiszylinders. Das Leitungselement 102 besteht aus einem Cermet. Das Cermet besteht aus einer Keramik und einem biokompatiblem Metall. Die Keramik des Cermet-Leitungselements 102 ist Aluminiumoxid und das Metall des Cermet-Leitungselements 102 ist Platin. Die Verbindung zwischen dem Leitungselement 102 und dem Grundkörper 101 ist co-gesintert und dadurch stoffschlüssig. Das Leitungselement 102 weist innerhalb der ersten Oberfläche 103 des Grundkörpers 101 einen ersten elektrisch leitfähigen Bereich 105 auf. Ferner weist das Leitungselement 102 innerhalb der zweiten Oberfläche 104 des Grundkörpers 101 einen zweiten elektrisch leitfähigen Bereich 106 auf. Der erste leitfähige Bereich 105 ist durch ein schichtartiges Kontaktelement 107 überdeckt. Ferner ist der zweite leitfähige Bereich 106 durch ein weiteres schichtartiges Kontaktelement 107 überdeckt. Dabei ist die Überdeckung der elektrisch leitfähigen Bereiche 105, 106 durch das Kontaktelement 107 im Wesentlichen randlos. Die Kontaktelemente 107 bestehen aus Gold. Die Kontaktelemente 107 wurden mit Hilfe einer elektrochemischen Abscheidung durch Reduktion von Gold-Kationen zu elementarem Gold erhalten. Die Kontaktelemente 107 weisen eine Porosität von 0,3 % auf und sind geschlossen porös.

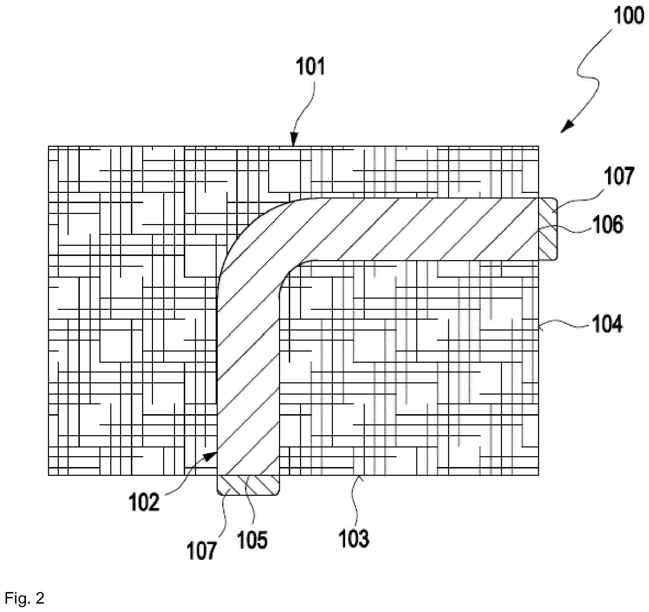

Figur 2 zeigt einen schematischen Querschnitt einer weiteren Ausführungsform einer erfindungsgemäßen Durchführung 100. In dieser Ausführungsform liegen die erste Oberfläche 103 und die zweite Oberfläche 104 des Grundkörpers 101 nicht gegenüber, sondern aneinandergrenzend über Eck. Die erste Oberfläche 103 ist die (gekrümmte) Mantelfläche einer Durchführung 100 mit der Grundform eines Kreiszylinders. Die zweite Oberfläche 104 bildet eine Seite der Grundflächen des Kreiszylinders. Das Leitungselement 102 erstreckt sich als gewundener Pfad von der ersten Oberfläche 103 des Grundkörpers 101 zur zweiten Oberfläche 104 des Grundkörpers 101. Das Kontaktelement 107 im gekrümmten Bereich der ersten Oberfläche 103 (Mantelfläche) überdeckt den leitfähigen Bereich 105 des Leitungselements 102 im Wesentlichen randlos. Die Kontaktelemente 107 bestehen aus Gold. Die Kontaktelemente 107 wurden mit Hilfe einer elektrochemischen Abscheidung durch Reduktion von Gold-Kationen zu elementarem Gold erhalten. Die Kontaktelemente 107 weisen eine Porosität von 0,3 % auf.

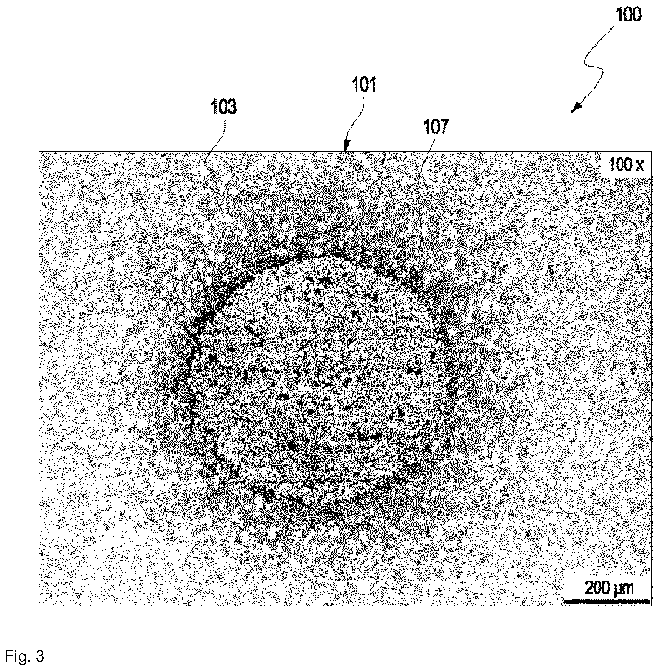

Figur 3 zeigt eine elektronenmikroskopische Aufnahme (Vergrößerung 50x) einer Draufsicht eines Ausschnitts einer erfindungsgemäßen Durchführung 100. Sichtbar ist die erste Oberfläche 103 des elektrisch isolierenden Grundkörpers 101. Innerhalb dieser Oberfläche 103 befinden sich vier erste elektrisch leitfähige Bereiche 105 (nicht sichtbar) von Leitungselementen 102 (nicht sichtbar). Die elektrisch leitfähigen Bereiche 105 sind vollständig und im Wesentlichen randlos durch die Kontaktelemente 107 überdeckt. Die Flächen der Kontaktelemente 107 sind kongruent mit den Flächen der elektrisch leitfähigen Bereiche 105. Die Kontaktelemente 107 bestehen aus Gold und weisen eine Porosität von 0,3 % auf.

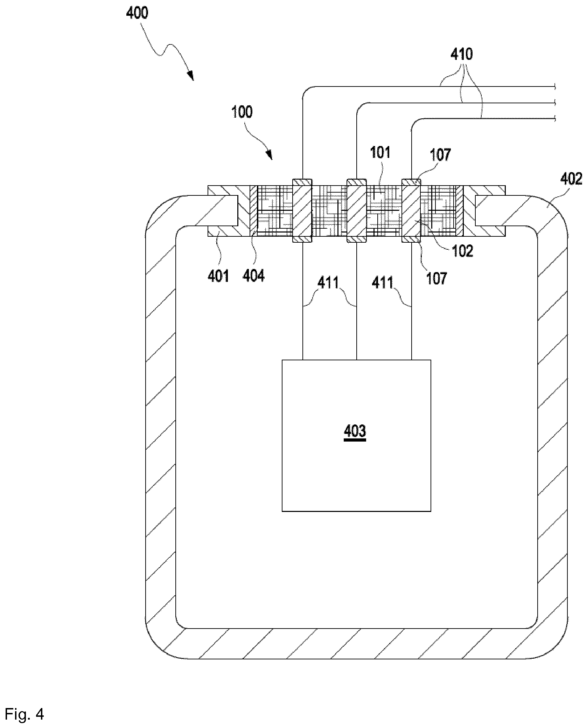

Figur 4 zeigt einen schematischen Querschnitt einer implantierbaren medizinischen Vorrichtung 400 beinhaltend eine erfindungsgemäße elektrische Durchführung 100. Bei der Vorrichtung 400 kann es sich beispielsweise um einen Herzschrittmacher oder einen Defibrillator handeln. Die Durchführung 100 weist einen elektrisch isolierenden Grundkörper 101 aus Aluminiumoxid auf. Der Grundkörper 101 beinhaltet drei Leitungselemente 102. Die Leitungselemente 102 bestehen aus einem Cermet. Das Cermet beinhaltet Aluminiumoxid und Platin. Die Leitungselemente 102 sind mit dem Grundkörper co-gesintert und dadurch stoffschlüssig und hermetisch mit dem Grundkörper 101 verbunden. Der Grundkörper 101 ist auf einer Umfangsfläche mit einem Flansch 101 hermetisch dicht verbunden. Die hermetisch dichte Verbindung zwischen Grundkörper 101 und Flansch 401 wird durch eine Lötverbindung 404 realisiert. Der Flansch 401 ist wiederum über eine Schweißverbindung hermetisch dicht mit einem Titangehäuse 402 der implantierbaren medizinischen Vorrichtung 400 verbunden. Die implantierbare medizinische Vorrichtung 400 beinhaltet einen Innenraum (Gehäuseseite) und eine Körperflüssigkeits-Seite oder Außenseite. Im Innenraum der Vorrichtung 400 befindet sich eine Mess-, Regel- und Steuerelektronik 403 und eine Batterie (nicht gezeigt). Die Durchführung 100 beinhaltet drei Kontaktelemente 107 auf der Außenseite und drei Kontaktelemente 107 auf der Innenseite der Vorrichtung. Die Kontaktelemente 107 auf der Außenseite sind jeweils mit Metalldrähten 410 verbunden. Die Verbindung zwischen Metalldrähten 410 und Kontaktelementen 107 ist eine Laserschweißverbindung. Die Metalldrähte 410 können beispielsweise mit dem IS4 Konnektor eines Anschlussterminals (nicht gezeigt) des Header-Blocks (nicht gezeigt) der Vorrichtung 400 verbunden werden. Die Kontaktelemente 107 auf der Innenseite sind jeweils mit Metalldrähten 411 verbunden. Die Verbindung zwischen Metalldrähten 411 und Kontaktelementen 107 ist eine Laserschweißverbindung. Die Metalldrähte 411 sind mit der Mess-, Regel- und Steuerelektronik 403 der implantierbaren Vorrichtung 400 verbunden.

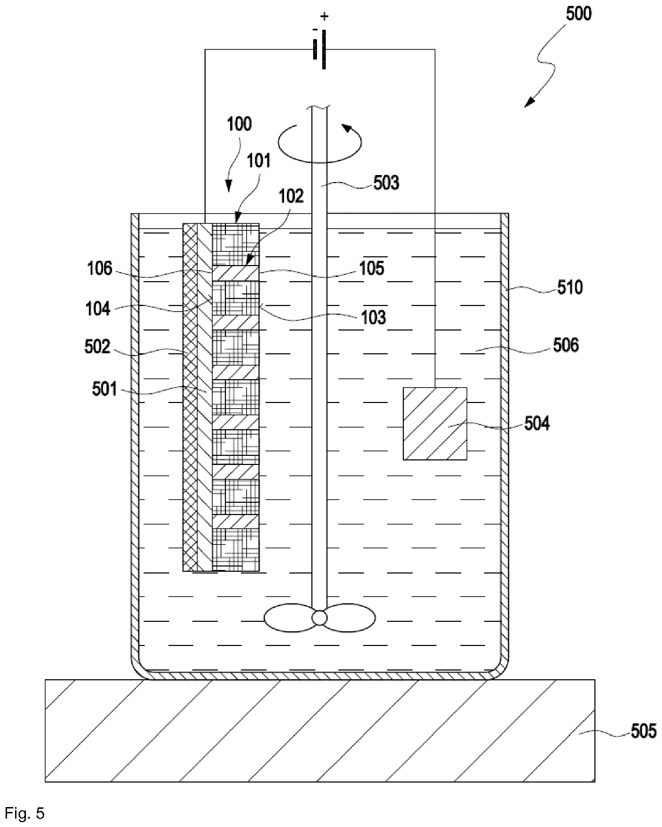

Figur 5 zeigt einen Versuchsaufbau 500 zur Durchführung des erfindungsgemäßen Verfahrens zur Herstellung der erfindungsgemäßen Durchführung 100. Gezeigt wird der Versuchsaufbau 500 vor Durchführung des erfindungsgemäßen Verfahrens. Die Durchführung weist zu diesem Zeitpunkt keine Kontaktelemente 107 auf. Die Durchführung 100 taucht in ein Aufnahmegefäß 510 ein. Das Aufnahmegefäß 510 ist beispielsweise ein Becherglas. In dem Aufnahmegefäß 510 befindet sich eine Elektrolytlösung 506. Im gezeigten Fall handelt es sich bei der Elektrolytlösung 506 um eine Gold-Elektrolytlösung auf Basis von Kalium-Gold-Cyanid. Während der Beschichtung der elektrisch leitfähigen Bereiche 105 der Durchführung 100 mit Gold wird die Elektrolytlösung 506 mit Hilfe eines Rührelements 503 bewegt. Gleichzeitig wird die Elektrolytlösung mit Hilfe eines Heizelements 505 auf ca. 80 °C geheizt. Die elektrische Durchführung beinhaltet eine Elektrodenschicht 501. Die Elektrodenschicht 501 kann aus mehreren Schichten bestehen. Im gezeigten Fall beinhaltet die Elektrodenschicht 501 eine Titanschicht und eine Goldschicht. Die Titanschicht dient der Haftvermittlung zwischen der Durchführung 100 und der Goldschicht. Die Elektrodenschicht 501 ist vollflächig von einer Isolierschicht 502 überlagert. Bei der Isolierschicht 502 handelt es sich um eine flexible Polymerschicht, die mit Hilfe eines Klebemittels mit der Elektrodenschicht 501 verbunden ist. Die Isolierschicht 502 verhindert die unbeabsichtigte und unnötige Beschichtung der Elektrodenschicht 501 mit Gold während des Beschichtungsprozesses. Die Elektrodenschicht 501 ist mit Hilfe einer Metallklemme (nicht gezeigt) als Kathode mit einer Gleichstromquelle (Minus-Pol) verbunden. Dadurch wird ein ausreichender Stromfluss zum leitfähigen Bereich 105 des Leitungselements 102 sichergestellt. In die Elektrolytlösung taucht außerdem eine Anode 504 ein, die ebenfalls mit der Gleichstromquelle verbunden ist (Plus-Pol). Die Anode ist ein platiniertes Titan-Streckmetall. Beim Anlegen von Gleichstrom bildet sich nahezu ausschließlich auf den leitfähigen Bereichen 105 der Durchführung 100 eine Goldschicht, die als Kontaktelement 107 der Durchführung 100 dient.

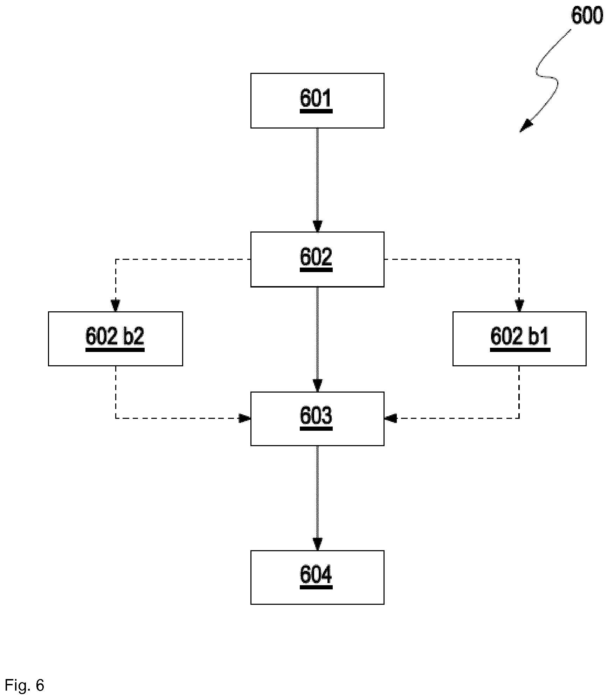

Figur 6 zeigt ein Ablaufdiagramm mit den Verfahrensschritten des erfindungsgemäßen Verfahrens 600. Das Verfahren beinhaltet die Verfahrensschritte 601 bis 604. Optional beinhaltet das Verfahren die Verfahrensschritte 602 b1 und/oder 602 b2. In Verfahrensschritt 601 wird die elektrische Durchführung 100 ohne Kontaktelemente 107 bereitgestellt. Die elektrische Durchführung 100 beinhaltet einen elektrisch isolierenden Grundkörper 101 und wenigstens ein elektrisch isolierendes Leitungselement 102. Das Leitungselement 102 beinhaltet ein Cermet. Grundkörper 101 und Leitungselement 102 sind co-gesinert. Das Leitungselement 102 erstreckt sich von einer ersten Oberfläche des Grundkörpers 103 zu einer zweiten Oberfläche des Grundkörpers 104. Das Leitungselement beinhaltet einen ersten elektrisch leitfähigen Bereich 105 innerhalb der ersten Oberfläche des Grundkörpers 103 und einen zweiten elektrisch leitfähigen Bereich 106 innerhalb der zweiten Oberfläche des Grundkörpers 104. Schritt 602 beinhaltet das Aufbringen einer Elektrodenschicht 501 auf die erste Oberfläche 103 des Grundkörpers 101. Die Elektrodenschicht 501 beinhaltet wenigstens ein Metall und kann aus mehreren Metallschichten bestehen. Die Elektrodenschicht wird durch einen Kathodenzerstäubungs-Prozess ("Sputtern") aufgebracht, wobei zur Haftvermittlung zunächst eine Titanschicht auf die erste Oberfläche des Grundkörpers 103 aufgebracht wird. Anschließend wird auf die Titanschicht durch einen weiteren Kathodenzerstäubungs-Prozess eine Goldschicht aufgebracht. Optional wird anschließend in Schritt 602 b1 eine Isolierschicht 502 auf die Elektrodenschicht 501 aufgebracht. Die Isolierschicht 502 beinhaltet ein Polymer und ein Klebemittel, mit dessen Hilfe die Isolierschicht 502 auf die Elektrodenschicht aufgebracht wird. Optional wird nach dem Aufbringen der Elektrodenschicht 501 gemäß Schritt 602 oder nach dem Aufbringen der Isolierschicht 502 gemäß Schritt 602b1 eine Reinigung der Oberflächen (Schritt 602 b2) der Durchführung 100 durch Eintauchen in eine alkalische Lösung und Schalten der Elektrodenschicht als Kathode durchgeführt. Diese Reinigung wird in Abhängigkeit der Beständigkeit der Isolierschicht gegenüber stark alkalischen Agenzien entweder nach oder vor dem Aufbringen der Isolierschicht 501 durchgeführt, wenn eine Isolierschicht für den Beschichtungsprozess verwendet wird. Im anschließenden Verfahrensschritt 603 wird die Durchführung 100 in eine Metall-Elektrolyt-Lösung 506 eingebracht und mittels elektrochemischer Abscheidung durch Reduktion von Metall-Kationen ein metallisches Kontaktelement 107 auf dem zweiten elektrisch leitfähigen Bereich 106 des Leitungselements 102 gebildet. Im letzten Verfahrensschritt 604 wird die Elektrodenschicht 501 gegebenenfalls zusammen mit der Isolierschicht 502 entfernt. Das Entfernen kann mechanisch durch Abschleifen oder chemisch durch orts-spezifisches oder selektives Ätzen erfolgen. Dadurch wird eine einseitig mit einem Kontaktelement 107 versehene Durchführung 100 erhalten. Optional kann das gesamte Verfahren wiederholt werden, um auch den ersten elektrisch leitfähigen Bereich 105 des Leitungselements 102 mit einem Kontaktelement 107 zu versehen. In diesem Fall darf es sich bei der Elektrodenschicht nicht um eine fest haftende Metallschicht handeln, da das Kontaktelement 107 auf dem zweiten elektrisch leitfähigen Bereich 106 des Leitungselements 102 mechanisch unversehrt bleiben sollte. Für diesen Fall ist eine angepresste Kupfer-Folie als Elektrodenschicht 501 geeignet.

### LISTE DER BEZUGSZEICHEN

- **100**: elektrische Durchführung
- **101**: elektrisch isolierender Grundkörper
- **102**: elektrisches Leitungselement
- **103**: erste Oberfläche des Grundkörpers
- **104**: zweite Oberfläche des Grundkörpers
- **105**: erster elektrisch leitfähiger Bereich des Leitungselements
- **106**: zweiter elektrisch leitfähiger Bereich des Leitungselements
- **107**: schichtartiges Kontaktelement
- **400**: implantierbare medizinische Vorrichtung
- **401**: Flansch
- **402**: Titangehäuse
- **403**: Elektronik
- **404**: Lötverbindung
- **410**: Metalldraht (Außenseite)
- **411**: Metalldraht (Gehäuseseite)
- **500**: Versuchsaufbau zum elektrochemischen Abscheiden
- **501**: Elektrodenschicht
- **502**: Isolierschicht
- **503**: Rührelement
- **504**: Anode
- **505**: Heizelement
- **506**: Elektrolytlösung
- **510**: Aufnahmegefäß
- **600**: erfindungsgemäßes Verfahren
- **601**: Verfahrensschritt a)
- **602**: Verfahrensschritt b)
- **602b1**: Verfahrensschritt b-1)
- **602b2**: Verfahrensschritt b-2)
- **603**: Verfahrensschritt c)
- **604**: Verfahrensschritt d)

## Patentansprüche

1. Elektrische Durchführung (100) für eine medizinisch implantierbare Vorrichtung (400), umfassend einen elektrisch isolierenden Grundkörper (101) und ein elektrisches Leitungselement (102), wobei das Leitungselement (102) ein Cermet beinhaltet, und wobei der Grundkörper (101) und das Leitungselement (102) durch eine stoffschlüssige gesinterte Verbindung verbunden sind, so dass das Leitungselement (102) hermetisch gegen den Grundkörper (101) abgedichtet ist; sich das Leitungselement (102) von einer ersten Oberfläche des Grundkörpers (103) durch den Grundkörper (101) hindurch zu einer zweiten Oberfläche des Grundkörpers (104) erstreckt, wobei das Leitungselement (102) einen ersten elektrisch leitfähigen Bereich (105) innerhalb der ersten Oberfläche des Grundkörpers (103) und einen zweiten elektrisch leitfähigen Bereich (106) innerhalb der zweiten Oberfläche des Grundkörpers (104) aufweist, und wenigstens einer der elektrisch leitfähigen Bereiche (105, 106) durch ein schichtartiges Kontaktelement (107), das ein Metall beinhaltet, wenigstens teilweise überlagert ist, so dass das Leitungselement (102) über das Kontaktelement (107) elektrisch leitend verbindbar ist, **dadurch gekennzeichnet, dass** das Kontaktelement (107) eine elektrochemisch erzeugte Schicht ist und das Kontaktelement (107) eine poröse Struktur aufweist, wobei
die Porosität des Kontaktelements (107) nicht mehr als 20 % beträgt.

2. Die elektrische Durchführung (100) nach Anspruch 1, **dadurch gekennzeichnet, dass** das Kontaktelement (107) den leitfähigen Bereich (105, 106) vollständig überlagert.

3. Die elektrische Durchführung (100) nach Anspruch 1 oder 2, **dadurch gekennzeichnet, dass** der Betrag der Fläche des schichtartigen Kontaktelements (107) nicht mehr als 25 % größer ist als der Betrag der Fläche des überlagerten leitfähigen Bereichs (105, 106).

4. Die elektrische Durchführung (100) nach einem der vorangehenden Ansprüche, **dadurch gekennzeichnet, dass** das Kontaktelement (107) eine mittlere Schichtdicke zwischen 1 µm und 50 µm aufweist.

5. Die elektrische Durchführung (100) nach einem der vorgenannten Ansprüche, **dadurch gekennzeichnet, dass** das Kontaktelement (107) eine Porosität von 0,1 bis 10 Vol.-% aufweist.

6. Die elektrische Durchführung (100) nach einem der vorangehenden Ansprüche, **dadurch gekennzeichnet, dass** das Kontaktelement (107) einen Porositätsgradienten aufweist.

7. Die elektrische Durchführung (100) nach Anspruch 6, **dadurch gekennzeichnet, dass** die mittlere Porosität des an das Leitungselement (102) angrenzenden Bereichs am niedrigsten ist und die mittlere Porosität mit zunehmendem Abstand von dem an das Leitungselement (102) angrenzenden Bereich ansteigt.

8. Die elektrische Durchführung (100) nach einem der vorangehenden Ansprüche, **dadurch gekennzeichnet, dass** die gemittelte Rautiefe Rz des schichtartigen Kontaktelements (107) zwischen 0,2 µm und 20 µm beträgt.

9. Verfahren zum Aufbringen von Kontaktelementen (107) auf elektrische Durchführungen (100), beinhaltend als Verfahrensschritte
a) Bereitstellen einer elektrischen Durchführung (100), wobei die Durchführung (100)
i. einen elektrisch isolierenden Grundkörper (101) und ein elektrisches Leitungselement (102) aufweist, wobei das Leitungselement (102) ein Cermet beinhaltet,
ii. der Grundkörper (101) und das Leitungselement (102) durch eine stoffschlüssige gesinterte Verbindung verbunden sind, so dass das Leitungselement (102) hermetisch gegen den Grundkörper (101) abgedichtet ist,
iii. sich das Leitungselement (102) von einer ersten Oberfläche des Grundkörpers (103) durch den Grundkörper hindurch zu einer zweiten Oberfläche des Grundkörpers (104) erstreckt,
iv. das Leitungselement (102) einen ersten elektrisch leitfähigen Bereich (105) innerhalb der ersten Oberfläche des Grundkörpers (103) und einen zweiten elektrisch leitfähigen Bereich (106) innerhalb der zweiten Oberfläche des Grundkörpers (104) aufweist,
b) Aufbringen einer elektrisch leitenden Elektrodenschicht (501) auf die erste Oberfläche des Grundkörpers (103), so dass eine leitfähige Verbindung zwischen dem ersten leitfähigen Bereich des Leitungselements (105) und der Elektrodenschicht (501) gebildet wird,
c) Einbringen der Durchführung (100) in eine Metall-Elektrolyt-Lösung (506) und Bilden eines metallischen Kontaktelements (107) im zweiten elektrisch leitfähigen Bereich (106) des Leitungselements (102) mittels elektrochemischer Abscheidung durch Reduktion von Kationen der Metall-Elektrolyt-Lösung,
wobei das Kontaktelement (107) eine poröse Struktur aufweist, wobei die Porosität des Kontaktelements (107) nicht mehr als 20 % beträgt,
d) Entfernen der Elektrodenschicht (501).

10. Das Verfahren nach Anspruch 9, **dadurch gekennzeichnet, dass** in einem weiteren Verfahrensschritt b-1) nach Schritt b) eine die Elektrodenschicht (501) überlagernde Isolierschicht (502) aufgebracht wird, so dass keine elektrochemische Abscheidung eines Metalls im Bereich der Elektrodenschicht (501) stattfindet.

11. Das Verfahren nach Anspruch 9 oder 10, **dadurch gekennzeichnet, dass** in einem weiteren Verfahrensschritt b-2) vor Durchführung von Schritt c) die elektrische Durchführung (100) in eine alkalische Lösung eingetaucht wird, wobei die Elektrodenschicht (501) mit einer Gleichstromquelle verbunden und als Kathode geschaltet wird, um die Oberflächen der elektrischen Durchführung zu reinigen.

12. Das Verfahren nach Anspruch 11, **dadurch gekennzeichnet, dass** die alkalische Lösung Natriumhydroxid und/oder Kaliumhydroxid beinhaltet und optional einen Zusatz eines Cyanid-Salzes beinhaltet.

13. Das Verfahren nach einem der Ansprüche 9 bis 12, **dadurch gekennzeichnet, dass** das Aufbringen der elektrisch leitenden Elektrodenschicht (501) in Schritt b) durch Anpressen eines elektrisch leitfähigen Polymers an die zweite Oberfläche erfolgt.

14. Das Verfahren nach einem der Ansprüche 9 bis 13, **dadurch gekennzeichnet, dass** die Elektrodenschicht (501) mehrschichtig ist, wobei der Mehrschicht-Aufbau eine leitfähige Paste und eine die leitfähige Paste überlagernde leitfähige Folie beinhaltet.

15. Das Verfahren nach einem der Ansprüche 9 bis 14, **dadurch gekennzeichnet, dass** die Stromdichte bei der elektrochemischen Abscheidung gemäß Schritt c) bezogen auf die Fläche des elektrisch leitfähigen Bereichs 0,1 bis 100 A/dm² beträgt, bevorzugt 0,5 bis 30 A/dm² beträgt, besonders bevorzugt 1 bis 15 A/dm² beträgt.

## Claims

1. Electrical feed-through (100) for a medically implantable device (400), comprising an electrically insulating base body (101) and an electrical conduction element (102), whereby the conduction element (102) includes a cermet, and whereby the base body (101) and the conduction element (102) are connected by a firmly bonded sintered connection such that the conduction element (102) is hermetically sealed with respect to the base body (101); whereby the conduction element (102) extends from a first surface of the base body (103) through the base body (101) to a second surface of the base body (104), whereby the conduction element (102) comprises a first electrically conductive region (105) within the first surface of the base body (103) and a second electrically conductive region (106) within the second surface of the base body (104), and at least one of the electrically conductive regions (105, 106) is covered, at least in part, by a layer-like contact element (107) that includes a metal such that the conduction element (102) can be connected in electrically conductive manner via the contact element (107), **characterised in that** the contact element (107) is an electrochemically-generated layer and **in that** the contact element (107) comprises a porous structure, whereby the porosity of the contact element (107) does not exceed 20%.

2. Electrical feed-through (100) according to claim 1, **characterised in that** the contact element (107) fully covers the conductive region (105, 106).

3. Electrical feed-through (100) according to claim 1 or 2, **characterised in that** the surface area of the layer-like contact element (107) is not more than 25% larger than the surface area of the covered conductive region (105, 106).

4. Electrical feed-through (100) according to any one of the preceding claims, **characterised in that** the contact element (107) has a mean layer thickness between 1 µm and 50 µm.

5. Electrical feed-through (100) according to any one of the preceding claims, **characterised in that** the contact element (107) comprises a porosity ranging from 0.1 to 10 vol. %.

6. Electrical feed-through (100) according to any one of the preceding claims, **characterised in that** the contact element (107) comprises a porosity gradient.

7. Electrical feed-through (100) according to claim 6, **characterised in that** the mean porosity of the region adjacent to the conducting element (102) is the lowest and **in that** the mean porosity increases with increasing distance from the region adjacent to the conduction element (102).

8. Electrical feed-through (100) according to any one of the preceding claims, **characterised in that** the average surface roughness Rz of the layer-like contact element (107) is between 0.2 µm and 20 µm.

9. Method for applying contact elements (107) onto electrical feed-throughs (100), containing, as procedural steps,
a) providing an electrical feed-through (100), whereby the feed-through (100)
i. comprises an electrically insulating base body (101) and an electrical conduction element (102), whereby the conduction element (102) includes a cermet,
ii. whereby the base body (101) and the conduction element (102) are connected by a firmly bonded sintered connection such that the conduction element (102) is hermetically sealed with respect to the base body (101),
iii. whereby the conduction element (102) extends from a first surface of the base body (103) through the base body to a second surface of the base body (104),
iv. whereby the conduction element (102) comprises a first electrically conductive region (105) within the first surface of the base body (103) and a second electrically conductive region (106) within the second surface of the base body (104),
b) applying an electrically conductive electrode layer (501) onto the first surface of the base body (103) such that a conductive connection is produced between the first conductive region of the conduction element (105) and the electrode layer (501),
c) introducing the feed-through (100) into a methyl-electrolyte solution (506) and forming a metallic contact element (107) in the second electrically conductive region (106) of the conduction element (102) by means of electrochemical deposition by reducing cations of the metal-electrolyte solution,
whereby the contact element (107) comprises a porous structure, whereby the porosity of the contact element (107) does not exceed 20%,
d) removing the electrode layer (501).

10. Method according to claim 9, **characterised in that** an insulating layer (502) covering the electrode layer (501) is applied in a further procedural step b-1) following step b), such that no electrochemical deposition of a metal in the region of the electrode layer (501) takes place.

11. Method according to claim 9 or 10, **characterised in that** the electrical feed-through (100) is immersed into an alkaline solution in a further procedural step b-2) before carrying out step c), whereby the electrode layer (501) is being connected to a direct voltage source and is being circuited as cathode in order to clean the surfaces of the electrical feed-through.

12. Method according to claim 11, **characterised in that** the alkaline solution includes sodium hydroxide and/or potassium hydroxide and optionally includes an added cyanide salt.

13. Method according to any one of the claims 9 to 12, **characterised in that** the application of the electrically conductive electrode layer (501) in step b) takes place by pressing an electrically conductive polymer to the second surface.

14. Method according to any one of the claims 9 to 13, **characterised in that** the electrode layer (501) is multi-layered, whereby the multilayer structure includes a conductive paste and a conductive foil superimposed on the conductive paste.

15. Method according to any one of the claims 9 to 14, **characterised in that** the current density during the electrochemical deposition according to step c), relative to the area of the electrically conductive region, is 0.1 to 100 A/cm², preferably 0.5 to 30 A/dm², particularly preferably 1 to 15 A/dm².

## Revendications

1. Traversée électrique (100) pour un dispositif implantable médicalement (400), comprenant un corps de base électriquement isolant (101) et un élément de conduction électrique (102), dans laquelle l'élément de conduction (102) contient un cermet, et dans laquelle le corps de base (101) et l'élément de conduction (102) sont connectés par une connexion frittée par liaison de matière de sorte que l'élément de conduction (102) est étanchéifié hermétiquement contre le corps de base (101) ; l'élément de conduction (102) s'étend d'une première surface du corps de base (103) à travers le corps de base (101) vers une seconde surface du corps de base (104), dans laquelle l'élément de conduction (102) présente une première région électriquement conductrice (105) au sein de la première surface du corps de base (103) et une seconde région électriquement conductrice (106) au sein de la seconde surface du corps de base (104), et au moins une des régions électriquement conductrices (105, 106) est au moins partiellement superposée par un élément de contact de type couche (107) qui contient un métal de sorte que l'élément de conduction (102) peut être connecté de manière électriquement conductrice par le biais de l'élément de contact (107), **caractérisée en ce que** l'élément de contact (107) est une couche générée par voie électrochimique et l'élément de contact (107) présente une structure poreuse, dans laquelle la porosité de l'élément de contact (107) ne se monte pas à plus de 20 %.

2. Traversée électrique (100) selon la revendication 1, **caractérisée en ce que** l'élément de contact (107) superpose entièrement la région conductrice (105, 106).

3. Traversée électrique (100) selon la revendication 1 ou 2, **caractérisée en ce que** le montant de la surface de l'élément de contact de type couche (107) n'est pas supérieur de plus de 25 % au montant de la surface de la région conductrice superposée (105, 106).

4. Traversée électrique (100) selon une des revendications précédentes, **caractérisée en ce que** l'élément de contact (107) présente une épaisseur de couche moyenne entre 1 µm et 50 µm.

5. Traversée électrique (100) selon une des revendications précédentes, **caractérisée en ce que** l'élément de contact (107) présente une porosité de 0,1 à 10 % en vol.

6. Traversée électrique (100) selon une des revendications précédentes, **caractérisée en ce que** l'élément de contact (107) présente un gradient de porosité.

7. Traversée électrique (100) selon la revendication 6, **caractérisée en ce que** la porosité moyenne de la région adjacente à l'élément de conduction (102) est au plus bas et la porosité moyenne augmente avec un écart croissant de la région adjacente à l'élément de conduction (102).

8. Traversée électrique (100) selon une des revendications précédentes, **caractérisée en ce que** la profondeur de rugosité moyennée de l'élément de contact de type couche (107) se situe entre 0,2 µm et 20 µm.

9. Procédé d'application d'éléments de contact (107) sur des traversées électriques (100), contenant en tant qu'étapes de procédé
a) mise à disposition d'une traversée électrique (100), dans lequel la traversée (100)
i. présente un corps de base électriquement isolant (101) et un élément de conduction électrique (102), dans lequel l'élément de conduction (102) contient un cermet,
ii. le corps de base (101) et l'élément de conduction (102) sont connectés par une connexion frittée par liaison de matière de sorte que l'élément de conduction (102) est étanchéifié hermétiquement contre le corps de base (101),
iii. l'élément de conduction (102) s'étend d'une première surface du corps de base (103) à travers le corps de base vers une seconde surface du corps de base (104),
iv. l'élément de conduction (102) présente une première région électriquement conductrice (105) au sein de la première surface du corps de base (103) et une seconde région électriquement conductrice (106) au sein de la seconde surface du corps de base (104),
b) application d'une couche d'électrodes électriquement conductrice (501) sur la première surface du corps de base (103) de sorte qu'une connexion conductrice entre la première région conductrice de l'élément de conduction (105) et la couche d'électrodes (501) est formée,
c) introduction de la traversée (100) dans une solution d'électrolyte métallique (506) et formation d'un élément de contact métallique (107) dans la seconde région électriquement conductrice (106) de l'élément de conduction (102) au moyen d'une précipitation électrochimique par réduction de cations de la solution d'électrolyte métallique, dans lequel l'élément de contact (107) présente une structure poreuse, dans lequel la porosité de l'élément de contact (107) ne se monte pas à plus de 20 %,
d) élimination de la couche d'électrodes (501).

10. Procédé selon la revendication 9, **caractérisé en ce que** dans une étape de procédé supplémentaire b-1) après l'étape b), une couche isolante (502) superposant la couche d'électrodes (501) est appliquée de sorte qu'aucune précipitation électrochimique d'un métal n'a lieu dans la région de la couche d'électrodes (501).

11. Procédé selon la revendication 9 ou 10, **caractérisé en ce que** dans une étape de procédé supplémentaire b-2) avant la réalisation de l'étape c), la traversée électrique (100) est immergée dans une solution alcaline, dans lequel la couche d'électrodes (501) est connectée à une source de courant continu et commutée en tant que cathode pour nettoyer les surfaces de la traversée électrique.

12. Procédé selon la revendication 11, **caractérisé en ce que** la solution alcaline contient de l'hydroxyde de sodium et/ou l'hydroxyde de potassium et contient en option un supplément d'un sel de cyanure.

13. Procédé selon une des revendications 9 à 12, **caractérisé en ce que** l'application de la couche d'électrodes électriquement conductrice (501) à l'étape b) s'effectue par pression d'un polymère électriquement conducteur sur la seconde surface.

14. Procédé selon une des revendications 9 à 13, **caractérisé en ce que** la couche d'électrodes (501) est multicouches, dans lequel la structure multicouches contient une pâte conductrice et un film conducteur superposant la pâte conductrice.

15. Procédé selon une des revendications 9 à 14, **caractérisé en ce que** la densité de courant lors de la précipitation électrochimique selon l'étape c) se monte à 0,1 à 100 A/dm² par rapport à la surface de la région électriquement conductrice, se monte de préférence à 0,5 à 30 A/dm², se monte de manière particulièrement préférée à 1 à 15 A/dm².
